(19) Europäisches Patentamt / European Patent Office / Office européen des brevets

(11) **EP 3 056 268 B1**

(12) **EUROPEAN PATENT SPECIFICATION**

(45) Date of publication and mention of the grant of the patent:
**22.03.2023 Bulletin 2023/12**

(21) Application number: **14852204.8**

(22) Date of filing: **09.10.2014**

(51) International Patent Classification (IPC):
**B01J 20/26** *(2006.01)*    **A61F 13/49** *(2006.01)*
**A61F 13/53** *(2006.01)*    **B01J 20/30** *(2006.01)*
**C08J 3/12** *(2006.01)*    **A61F 13/15** *(2006.01)*

(52) Cooperative Patent Classification (CPC):
**A61F 13/53; A61F 13/15658; B01J 20/261; B01J 20/267;** A61F 2013/530481; B01J 2220/68

(86) International application number:
**PCT/JP2014/077102**

(87) International publication number:
**WO 2015/053372 (16.04.2015 Gazette 2015/15)**

(54) **PARTICULATE WATER ABSORBER COMPRISING WATER-ABSORBING RESIN AS MAIN COMPONENT AND PROCESS FOR MANUFACTURING SAME**

PARTIKELFÖRMIGER WASSERABSORBER MIT WASSERABSORBIERENDEM HARZ ALS HAUPTKOMPONENTE UND VERFAHREN ZUR HERSTELLUNG DAVON

ABSORBEUR D'EAU PARTICULAIRE COMPRENANT UNE RÉSINE D'ABSORPTION D'EAU EN TANT QUE CONSTITUANT PRINCIPAL ET SON PROCESSUS DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2013 JP 2013212145**
             **30.05.2014 JP 2014112143**

(43) Date of publication of application:
**17.08.2016 Bulletin 2016/33**

(73) Proprietor: **Nippon Shokubai Co., Ltd. Osaka-shi, Osaka 541-0043 (JP)**

(72) Inventors:
• **KATADA, Yoshiki**
  **Himeji-shi**
  **Hyogo 671-1282 (JP)**
• **ISHIZAKI, Kunihiko**
  **Himeji-shi**
  **Hyogo 6711282 (JP)**

(74) Representative: **Glawe, Delfs, Moll Partnerschaft mbB von Patent- und Rechtsanwälten Postfach 13 03 91 20103 Hamburg (DE)**

(56) References cited:
WO-A1-2010/079075    WO-A1-2011/040530
WO-A1-2013/073614    JP-A- 2000 007 790
JP-A- 2006 057 075    US-A1- 2009 275 470
US-A1- 2012 189 861

EP 3 056 268 B1

**Description**

TECHNICAL FIELD

The present invention relates to a particulate water-absorbing agent containing a water-absorbing resin as a main component and a method for producing same, which is preferably used for absorbent articles such as disposable diapers, sanitary napkins and incontinence pads.

BACKGROUND ART

In recent years, for absorbent articles such as disposable diapers, sanitary napkins and incontinence pads, a water-absorbing resin is widely used as its constituent material for the purpose of absorbing a body fluid such as urine and menstrual blood. As to the foregoing water-absorbing resin, for instance, partially neutralized crosslinked polyacrylic acid; a hydrolyzed starch-acrylic acid graft polymer; a saponified vinyl acetate-acrylic acid ester copolymer; a hydrolyzed copolymer of: acrylonitrile or acrylamide, or its crosslinked product; and a crosslinked polymer of cationic monomers are known.

When a water-absorbing resin is used as an absorbent material for disposable diapers, after absorbing urine, the water-absorbing resin can get degraded over time and lose its liquid permeability and gel strength, causing a problem of urine leakage from the disposable diapers. Specifically, such a problem has been pointed out to be an issue over gel stability.

As a means of improving gel stability of said water-absorbing resin, various additives for a water-absorbing resin are suggested, and the following: a method for adding a reducing agent (Patent Literatures 1 to 3); a method for adding a reducing agent and an anti-oxidizing agent (Patent Literature 4); a method for adding an oxidizing agent (Patent Literature 5); a method for adding an anti-oxidizing agent (Patent Literature 6); a method for adding a chain-transfer agent to monomers in polymerization (Patent Literature 7); a method for adding a chelating agent (Patent Literatures 8 to 13) and the like, are suggested. Because this type of deterioration of a water-absorbing resin, due to urine in particular, is believed to be caused by the presence of a trace amount of metal ions, a technique to improve gel stability of the water-absorbing resin by adding so-called chelating agent to a water-absorbing resin so as to trap metal ions and suppress generation of radical species has been suggested.

As a means of improving gel stability of said water-absorbing resin, for instance, a technique of adding a chelating agent to monomers (Patent Literature 8); a method for adding a chelating agent to a hydrogel of a water-absorbing resin and drying same (Patent Literature 9); a method for adding a chelating agent when mixing a water-absorbing resin with an aqueous solution of a surface crosslinking agent (Patent Literature 10); a method for adding an aqueous solution of a chelating agent after surface crosslinking a water-absorbing resin and granulating same (Patent Literatures 11 and 12); and a method for adding a chelating agent to fine powder generated during a production step of a water-absorbing resin for reuse (Patent Literature 13) and the like are disclosed.

Further, a chelating agent is also used for: the prevention of coloration of a water-absorbing resin (Patent Literatures 14 to 16 and 19 to 21) as well as the removal of odors from disposable diapers, in addition to the prevention of deterioration of a water-absorbing resin due to urine.

Furthermore, also disclosed are: a method for granting light resistance, coloration resistance and deodorizing properties by reducing impurities in aminoacetic acid based chelating agents (Patent Literature 17); and a method for granting both gel stability and coloration resistance by simultaneously using an aminocarboxylic acid based chelating agent, an oxygen-containing reducing inorganic salt and an organic anti-oxidizing agent (Patent Literature 18).

Further, a water-absorbing resin is known to have different kinds of residual monomers and a different degree of liquid permeability depending upon its particle sizes (Patent Literatures 22 and 23). WO 2006/033477 also discloses a particulate water-absorbing agent with a chelating agent and the use for absorbent articles.

CITATION LIST

PATENT LITERATURES

Patent Literature 1: US Patent No. 4,959,060

Patent Literature 2: US Patent No. 4,863,989

Patent Literature 3: EP Patent No. 1477349

Patent Literature 4: US Patent No. 4,972,019

Patent Literature 5: JP Publication No.S63-153060

Patent Literature 6: JP Unexamined Patent Publication No. S63-127754

Patent Literature 7: US Patent No. 6,335,406

Patent Literature 8: JP Unexamined Patent Publication No. 2000-038407

Patent Literature 9: JP Unexamined Patent Publication No. H11-246674

Patent Literature 10: JP Unexamined Patent Publication No. H11-315148

Patent Literature 11: JP Unexamined Patent Publication No. H11-315147

Patent Literature 12: US Patent No. 7,473,470

Patent Literature 13: JP Unexamined Patent Publication No. 2000-007790

Patent Literature 14: JP Patent No. 3107873

Patent Literature 15: JP Unexamined Patent Publication No. 2003-206381

Patent Literature 16: US Patent No. 7,737,231

Patent Literature 17: JP Unexamined Patent Publication No. 2001-234087

Patent Literature 18: EP Patent No. 1645596

Patent Literature 19: EP Patent No. 2112172

Patent Literature 20: EP Patent No. 2163302

Patent Literature 21: WO Publication No. 2011/040530

Patent Literature 22: US Patent No. 8,182,916

Patent Literature 23: US Patent No. 5,599,335

## SUMMARY OF THE INVENTION

### TECHNICAL PROBLEMS

Even when gel stability or the like is improved by way of the foregoing means, evaluation of gel stability of water-absorbing agents removed from absorbent articles, which have water-absorbing resins as a main component, such as disposable diapers and the like, show that gel stability of a water-absorbing agent can vary amongst absorbing articles, and a problem that gel stability has not achieved its optimal level remains. A decline in gel stability is significant especially in the case of absorbent articles, where particles of fine particle sizes are largely concentrated.

Further, in the above Patent Literatures 8 to 21, a method for adding a chelating agent: to monomers at the time of polymerization; to a hydrogel-forming crosslinked polymer prior to drying; at the time of surface crosslinking; at the time of granulation; and at the time of collection of granules is disclosed. In such methods, a chelating agent is evenly added as per each particle size. However, there is a problem that a degree of variation in gel stability of the obtained water-absorbing agents is magnified.

The present invention is devised in view of the foregoing problems in the art, and one objective of the present invention is to provide a water-absorbing agent having high gel stability, little variation in gel stability amongst different particle sizes, and good water-absorbing properties (for example, Absorbency Against Pressure, Centrifuge Retention Capacity, saline flow conductivity, anti-blocking property after moisture absorption, low coloration, or the like), and a method for producing such a water-absorbing agent.

SOLUTION TO PROBLEMS

To solve the foregoing problems, the inventors of the present invention conducted extensive studies and found that variation in gel stability amongst absorbent articles such as disposable diapers or the like was due to the fact that the likelihood of deterioration of a water-absorbing resin varies, depending upon a particle size.

To be more specific, for a particulate water-absorbing agent, particles with small particle size can be more easily deteriorated than particles with large particle size, as they have a larger surface area to be in contact with urine, requiring more chelating agent to be included.

Also, when a particulate water-absorbing agent is incorporated into an absorbent article, or in particular, when a predetermined amount of a water-absorbing resin (5 to 20 g per diaper) is incorporated into a disposable diaper, variation in particle size within each diaper, as well as variation in particle size depending on a location in a single diaper (a central portion, a peripheral portion, top and bottom portions in a thickness direction of a disposable diaper) can cause variation in gel stability.

Accordingly, in supplying a powdered-form particulate water-absorbing agent to an absorbent article from an apparatus to produce absorbent articles such as disposable diapers and the like, if the particulate water-absorbing agent with its fine particle size is too concentrated, there is a problem of the decline of gel stability even if a chelating agent is added in its predetermined amount. In other words, it was discovered that gel stability would vary even when a chelating agent is added evenly as per each different particle size.

In view of the above, to control varying gel stability amongst particles in a particulate water-absorbing agent, the inventors of the present application adjusted an amount of the chelating agents to be added in a way that satisfies Formula (1), instead of evenly adding as per each particle size, so as to obtain a particulate water-absorbing agent having high gel stability and few variations of the gel stability amongst particles in the particulate water-absorbing agent.

To be more specific, to solve the foregoing problems the first embodiment of the present invention provides a particulate water-absorbing agent comprising a water-absorbing resin as a main component and a chelating agent, the water-absorbing resin containing particles with a particle diameter of less than 300 $\mu$m and particles with a particle diameter of 300 $\mu$m or more but less than 850 $\mu$m, as specified by a Japanese Industrial Standard (JIS) standardized sieve, wherein an amount of the chelating agent included is 0.001 to 5 parts by weight per 100 parts by weight of a water-absorbing resin in solid and satisfies Formula (1):

[Mathematical 1]

$$1.2 < C1/C2 < 4.0 \ ... \ (1)$$

where, in Formula (1), C1 indicates that an amount (ppm) of the chelating agent present in the particles of a particle diameter of less than 300 $\mu$m, as specified by a JIS standardized sieve, amongst particle components for the particulate water-absorbing agent, and C2 indicates that an amount (ppm) of the chelating agent present in the particles of a particle diameter of 300 $\mu$m or more but less than 850 $\mu$m, as specified by a JIS standardized sieve, amongst particle components for the particulate water-absorbing agent.

The second embodiment of the present invention provides a method for producing a particulate water-absorbing agent comprising a water-absorbing resin as a main component and a chelating agent, the water-absorbing resin containing particles with a particle diameter of less than 300 $\mu$m and particles with a particle diameter of 300 $\mu$m or more but less than 850 $\mu$m, as specified by a Japanese Industrial Standard (JIS) standardized sieve, wherein an amount of the chelating agent included is 0.001 to 5 parts by weight per 100 parts by weight of a water-absorbing resin in sold and satisfies Formula (1):

[Mathematical 2]

$$1.2 < C1/C2 < 4.0 \ ... \ (1)$$

where, in Formula (1), C1 indicates that an amount (ppm) of the chelating agent present in the particles of a particle diameter of less than 300 $\mu$m, as specified by a JIS standardized sieve, amongst particle components for the particulate water-absorbing agent, and C2 indicates that an amount (ppm) of the chelating agent present in the particles of a particle diameter of 300 $\mu$m or more but less than 850 $\mu$m, as specified by a JIS standardized sieve, amongst particle components for the particulate water-absorbing agent; and wherein the method comprises one of the following a) and b):

   a) the steps of:adding a chelating agent to a surface-crosslinked water-absorbing resin to obtain a water-absorbing resin granule, of which weight average particle diameter (D50) is increased to 1.01 times to 10 times relative to the weight average particle diameter before adding the chelating agent; and crushing the water-absorbing resin granule and reducing the weight average particle diameter (D50) by 5% or more to obtain a particulate water-absorbing agent; and

b) the steps of: sizing a surface-crosslinked water-absorbing resin into a plurality of different particle sizes; adding a chelating agent as per the plurality of the different particle sizes; and remixing a plurality of the water-absorbing resins, to which said chelating agent was added, to obtain a particulate water-absorbing agent.

The third embodiment provides for an absorbent article comprising a particulate water-absorbing agent as set forth in any one of claims 1 to 14.

ADVANTAGEOUS EFFECT OF THE INVENTION

According to the embodiment of the present invention, a particulate water-absorbing agent having high gel stability, little variation in gel stability amongst different particle sizes, and good water-absorbing properties (for example, Absorbency Against Pressure, Centrifuge Retention Capacity, saline flow conductivity, anti-blocking property after moisture absorption, low coloration, or the like) can be obtained.

DESCRIPTION OF EMBODIMENTS

Description regarding the embodiments of the present invention is provided below. Specifically, the present invention is not intended to be limited to each embodiment described below, and can be modified into various embodiments within the scope illustrated by the claims. Exemplary embodiments that can be obtained by appropriately combining the technical means that are respectively disclosed in different exemplary embodiments are also included in the technical scope of the present invention.

Definitions of Terms

(1-1) "Water-absorbing resin" and "water-absorbing agent"

In the embodiment of the present invention, the term "water-absorbing resin" means a polymer gelling agent for aqueous matter and is water-swellable and water-insoluble, and has the physical properties described below. Specifically, it is intended to be the polymer gelling agent having the following properties: for "water swellability", CRC (Centrifuge Retention Capacity; fluid retention capacity without pressure) of 5 (g/g) or higher as defined in ERT 441.2-02 (2002), and for "water insolubility", Ext (Extractable; soluble amount) of 50% by weight or less as defined in ERT 470.2-02 (2002). The water-absorbing resin may be appropriately designed according to its use and can be, but not limited to, a hydrophilic crosslinked polymer, where carboxyl group-containing unsaturated monomers are polymerized and crosslinked.

Further, in the embodiment of the present invention, the "water-absorbing agent" means an absorbent gelling agent having the foregoing water-absorbing resin as a main component. The term "main component" indicates a condition where an amount contained is: at least 50% by weight or more, preferably at least 70% by weight or more, more preferably at least 80% by weight or more, and even more preferably at least 90% by weight or more. Thus, said water-absorbing agent is not limited to a formation where the polymers make up the entire amount (100% by weight), and as long as the foregoing physical properties (CRC, Ext) are satisfied, it is preferably a composition that contains additives. Furthermore, the "aqueous matter" may be a gas, liquid or solid, and as long as water is contained, it may be either water, aqueous solution or aqueous dispersion. Specific examples include: urine, blood, menstrual blood, sea water and various kinds of waste water.

For a form of the foregoing water-absorbing resin and the water-absorbing agent, it can be, without limiting, in the form of sheet, fiber, film, gel and various others, but in the embodiment of this invention, preferably in the form of particle for ease of handling.

Meanwhile, in the embodiment of the present invention, intermediates generated in the process of producing a water-absorbing resin (for example, hydrogel-forming crosslinked polymers after polymerization process, dry polymers after drying process, water-absorbing resin granules prior to surface crosslinking process and the like) are collectively referred to as a "water-absorbing resin". On the other hand, if some additives or the like are contained, such a composition is referred to as a "water-absorbing agent". For a form of a final product obtained according to the embodiment of the present invention, particulate form is preferred, hence a particulate water-absorbing agent.

(1-2) "Polyacrylic acid (salt))"

In the embodiment of the present invention, the term "polyacrylic acid (salt)" indicates polyacrylic acid and/or polyacrylic acid salt, which optionally includes a graft component, and it means a polymer having acrylic acid and/or acrylic acid salt (hereinafter referred to as "acrylic acid (salt)") as a repeat unit. Said "main component" means a condition in which a portion (amount) of acrylic acid (salt) used is, in general, 50 to 100 Mol% of the entire monomers (not including internal crosslinkers) used in polymerization (excluding the internal cross-linking agent), preferably 70 to 100 Mol%, more preferably 80 to 100 Mol%, even more preferably 90 to 100 Mol%, or substantially 100 Mol%. In addition, polyacrylic acid salt as a polymer essentially includes a water soluble salt, and preferably includes a monovalent salt, and more preferably includes an alkali metal salt or an ammonium salt.

(1-3) "EDANA" and "ERT"

The term "EDANA" is an abbreviation for the European Disposables and Nonwovens Associations, and the term "ERT" is an abbreviation for EDANA Recommended Test Methods, European standard (meaning essentially world standard) test methods for superabsorbent resins.
In the embodiment of the present invention, unless specifically stated otherwise, the physical properties of water-absorbing resins were measured in accordance with the original ERT (Revised in 2002, public art). Also, the physical properties of a particulate water-absorbing agent according to the embodiment of the present invention were measured likewise.

(1-3-1) "CRC" (ERT441.2-02)

The term "CRC" is an abbreviation for Centrifuge Retention Capacity, meaning fluid retention capacity without pressure (it can also be called "absorption rate"). To be more specific, CRC is a fluid retention capacity (unit; g/g) obtained after 0.2 g of a water-absorbing resin or a particulate water-absorbing agent was placed in a non-woven fabric bag and allowed to soak in a large excess of a 0.9% by weight of an aqueous sodium chloride solution for 30 minutes for free swelling, followed by drying in a centrifuge (250 G). In the embodiment of the present invention, the evaluation was made on values including corrections for moisture content.

(1-3-2) "AAP" (ERT442.2-02)

The term "AAP" is an abbreviation for Absorption Against Pressure, meaning fluid retaining capacity under pressure. Specifically, AAP is a fluid retention capacity (unit: g/g) obtained after 0.9 g of a water-absorbing resin or a particulate water-absorbing agent was allowed to swell in a large excess of 0.9% by weight of an aqueous sodium chloride solution for one hour under a pressure of 2.06 kPa (0.3 psi).
According to ERT442.2-02, it is listed as "Absorption Under Pressure", the foregoing AAP means essentially the same. Furthermore, in the embodiment of the present invention, a measurement can be made after modifying the condition for pressure to be 4.83 kPa (0.7 psi), and a measurement result obtained under such condition is indicated as AAP 4.83 kPa (AAP measured under 2.06 kPa is indicated as AAP 2.06 kPa). In addition, the evaluation was made on values including corrections for moisture content.

(1-3-3) "Ext" (ERT470.2-02)

The term "Ext" is an abbreviation for Extractables, meaning an amount for soluble content. Specifically, Ext is a value (unit:% by weight) obtained after 1.0 g of a water-absorbing resin or a particulate water-absorbing agent is added to 200 ml of 0.9% by weight of an aqueous sodium chloride solution and stirred for 16 hours at 500 rpm, and then a value (unit; % by weight) for the amount of polymers dissolved was measured through pH titration.
In the embodiment of the present invention, the measurement was taken by modifying the stirring time to 1 hour. Further, the evaluation was made on values including corrections for moisture content.

(1-3-4) "Moisture Content" (ERT430.2-02)

The "moisture content" means an amount of moisture in a water-absorbing resin or a particulate water-absorbing agent. Specifically, for 4.0g of a water-absorbing resin or a particulate water-absorbing agent, it is a value computed based upon an amount lost from drying for 3 hours at 105°C (unit; % by weight). In the embodiment of the present invention, the measurement was made by modifying a sample amount to 1.0 g and a drying temperature to 180°C.

(1-3-5) "PSD" (ERT420.2-02)

"PSD" is an abbreviation for Particle Size Distribution, meaning a particle size distribution measured by sieve classification. Meanwhile, a weight average particle diameter (D50) and logarithmic standard deviation for particle size distribution ($\sigma\zeta$) are measured in the same manner as indicated in "(3) Mass-Average Particle Diameter (D50) and Logarithmic Standard Deviation ($\sigma\zeta$) of Particle Diameter Distribution", which is described in the U.S. Patent No. 7,638,570. In the embodiment of the present invention, an amount and other measurements of a chelating agent according to particle sizes were obtained using the method described above.

(1-4) Others

In this specification, the expression for a range, "X to Y", means "X or more and Y or less". Also, unless otherwise noted, a unit for weight, "t (ton)" means "Metric ton", and "ppm" means "ppm by weight" or "ppm by mass". Further, "weight" and "mass", "parts by weight" and "parts by mass", and "% by weight" and "% by mass", respectively, are used as synonyms. In addition, "-acid (salt)" means "-acid and/or salt", and "(meth)acrylic" means "acrylic and/or methacrylic", respectively.

Method for producing polyacrylic acid (salt)-based water-absorbing resin

Hereinafter, described is a method for producing a polyacrylic acid (salt)-based water-absorbing resin, which is used for a particulate water-absorbing agent according to the embodiment of the present invention.

(2-1) Step for preparing aqueous monomer solution

The present step is one for producing an aqueous solution containing acrylic acid (salt) as a main component (hereinafter referred to as an "aqueous monomer solution"). Here, the "main component" means the same as the contents described in (1-2) "Polyacrylic acid (salt))". Further, instead of the aqueous monomer solution, a slurry of monomers can be used within the extent in which the fluid retention capacity is not lowered, although the below is described using an aqueous monomer solution for simplicity.

(Acrylic acid)

From the viewpoint of the effectiveness of the embodiment of the present invention, acrylic acid is used as a monomer, yet acrylic acid such as this usually contains a trace amount of polymerization inhibitors, impurities and the like.
As for a polymerization inhibitor, preferably phenols, more preferably methoxyphenols, and even more preferably p-methoxyphenols can be given, and their concentrations are, from the viewpoint of polymerization inhibition effect and color of a water-absorbing resin or a particulate water-absorbing agent to be obtained, preferably 1 to 200 ppm, and more preferably 10 to 160 ppm. Further, as for the foregoing impurities, a reference is made to the U.S. Patent Application Publication No. 2008/0161512 and the like.

(Basic compositions)

A main component of a water-absorbing resin or a particulate water-absorbing agent obtained through the embodiment of the present invention is polyacrylic acid (salt), in which acrylic acid (salt) is polymerized and crosslinked. To obtain said polyacrylic acid (salt), it is preferable to neutralize acrylic acid with a basic composition. Here, the "basic composition" means a composition containing a basic compound.
As examples of the basic compounds, carbonate (hydrogen) salt of an alkali metal, alkali metal hydroxides, ammonia, organic amine and the like are included. In particular, from the viewpoint of obtaining a water-absorbing resin or a particulate water-absorbing agent with even better physical properties, alkali metal hydroxides such as sodium hydroxide, potassium hydroxide and lithium hydroxide are preferred, and even more preferable is sodium hydroxide.

(Neutralization process)

A neutralization process in this step (or it may also be referred to as a neutralization step) encompasses neutralization of acrylic acid as a monomer, or neutralization of a hydrogel forming crosslinked polymer (hereinafter referred to as "hydrogel"), which is obtained through polymerizing and crosslinking acrylic acid (post-neutralization). In either case, it can be done on a batch basis or a continuous basis, but preferably a continuous basis.
For preferable conditions for neutralization, including basic composition, temperature retention time, device and the like, the neutralization conditions as described in International Publication No. 2009/123197 and U.S. Patent Application Publication No. 2008/0194863 are applied.

A rate of neutralization according to the embodiment of the present invention is preferably 10 to 90 mol%, more preferably 40 to 85 mol%, even more preferably 50 to 80 mol%, and yet even more preferably 60 to 75 mol%, with respect to an acid group in a monomer aqueous solution or in post-polymerization hydrogel.

If the rate of neutralization is less than 10 mol%, the fluid retention capacity without load (CRC) significantly decreases. On the other hand, if the rate of neutralization exceeds 90 mol%, it is possible that a water-absorbing resin with good fluid retention capacity under load (AAP) may not be obtained.

This can be said for neutralization following polymerization (subsequent neutralization), or for a rate of neutralization for a particulate water-absorbing agent as a final product.

Further, although acrylic acid salt is essentially a monovalent salt, it can be neutralized as a polyvalent metal salt if in an extremely small amount (for example, approximately 5 mol%).

(Internal crosslinking agent)

Examples of an internal crosslinking agent that can be used in the embodiment of the present invention include a compound having two or more substituent groups that could react with acrylic acid. To be more specific, one or more species out of the internal crosslinking agent(s) described in the 14th column of the U.S. Patent No. 6,241,928 are used.

Meanwhile, from the viewpoint of fluid retention capacity of a water-absorbing resin or a particulate water-absorbing agent to be obtained, the following are used: preferably a compound having two or more polymerizable unsaturated groups, more preferably compound having thermal decomposability at around a drying temperature described below, even more preferably a compound having two or more polymerizable unsaturated groups in (poly)alkylene glycol structural unit (namely, an allyl group and a (meth)acrylate group, but especially preferably acrylate group).

More specifically, it is preferable to use (poly)alkylene glycol di(meth)acrylate or tri(meth)acrylate at polymerization. As for an alkylene glycol unit, a polyethylene glycol unit where a number of n in an internal crosslinking agent is preferably 1 to 100, and more preferably 6 to 50 are used.

In the embodiment of the present invention, the amount used for the foregoing internal crosslinking agent is preferably 0.005 to 2 mol%, more preferably 0.01 to 1 mol%, and even more preferably 0.05 to 0.5 mol% per a monomer. By setting the amount used for the internal crosslinking agent to be these ranges mentioned above, desirable water-absorbing properties can be achieved.

Aside from the aforementioned method of crosslinking in which the internal crosslinking agent is added prior to polymerization, a method of adding the internal crosslinking agent (preferably a polyglycidyl compound) during or after polymerization and conducting subsequent crosslinking, a method of radical crosslinking using a radical polymerization and a method of crosslinking using radiation such as electron beam and the like can be employed.

In particular, a method for preparing an aqueous monomer solution in which a predetermined amount of an internal crosslinking agent is added, and crosslinking concurrently with polymerization of said aqueous monomer solution is preferable. Meanwhile, if needed, an internal crosslinking agent (preferably a polyglycidyl compound) may be added during or after polymerization.

(Other monomers used in combination)

According to the embodiment of the present invention, it is possible to use another monomer besides acrylic acid (salt) (hereinafter referred to as "another monomer"). An example of such a monomer includes a water soluble unsaturated monomer or a hydrophobic unsaturated monomer.

To be more specific, one or more species of monomers described in Paragraph [0035] of the U.S. Patent Application Publication No. 2005/215734 (yet excluding acrylic acid) are used depending upon a purpose.

Meanwhile, a particulate water-absorbing agent according to the embodiment of the present invention encompasses one having said water soluble unsaturated monomer or hydrophobic unsaturated monomer as a copolymerization component.

(Other substances added to an aqueous monomer solution)

In the embodiment of the present invention, the following substance can be added at the time of preparing an aqueous monomer solution besides the aforementioned substances.

To be more specific, for the purpose of improving various physical properties associated with a water-absorbing resin or a particulate water-absorbing agent, preferably 50% by weight or less, or more preferably 20% by weight or less of a water soluble resin or a water-absorbing resin can be added, or various kinds of foaming agents (such as a carbonate, an azo compound and an air bubble), a surfactant, a chain transfer agent and the like can be added at preferably 5% by weight or less, and at more preferably 1% by weight or less. Those substances can take a formation of being added to an aqueous monomer solution or being added during polymerization.

While a use of the water soluble resin or the water-absorbing resin generates a graft polymer or a water-absorbing resin composition (for example, a starch-acrylic acid polymer, a PVA-acrylic acid polymer and the like), these polymers, including the water-absorbing resin composition, are called "polyacrylic acid (salt)-based water-absorbing resin" in the embodiment of the present invention. Further, the particulate water-absorbing agent according to the embodiment of the present invention can also be made using these polymers and a water-absorbing resin composition.

(Concentration of a monomer component)

In the present step, an aqueous monomer solution is prepared by mixing each substance described above. In so doing, a concentration of a monomer component in an aqueous monomer solution is, but not limited to, preferably 10 to 80% by weight, more preferably 20 to 75% by weight, and even more preferably 30 to 70% by weight, from the viewpoint of physical properties for a water-absorbing resin or a particulate water-absorbing agent.

Further, when aqueous solution polymerization or reverse phase suspension polymerization is employed, a solvent other than water can be additionally used, if needed. A type of the solvent used is not to be limited.

Meanwhile, said "concentration of a monomer component" is a value that can be obtained by Formula (3), and the aqueous monomer solution does not include a graft component, a water-absorbing resin and a hydrophobic solvent for a reverse phase suspension polymerization.

[Mathematical 3]

$$\text{Concentration of Monomer Component (\% by weight)} = \text{(Weight of Monomer Component)}/\text{(Weight of Aqueous Monomer Solution)} \times 100 \ \dots \ (3)$$

(2-2) Polymerization step

This is a step for obtaining a hydrogel-forming crosslinked polymer (hereinafter referred to as "hydrogel") through polymerization of the acrylic acid (salt)-based aqueous monomer solution obtained from the aforementioned step for preparing the aqueous monomer solution.

(Polymerization initiator)

The polymerization initiator employed in the embodiment of the present invention is selected in accordance with a form of polymerization and the like, including, for instance, a thermal decomposition polymerization initiator and a photolysis polymerization initiator, or a redox polymerization initiator, which concurrently uses a reducing agent that facilitates decomposition of these polymerization initiators.

To be more specific, out of all the initiators indicated in the 5th column of the U.S. Patent No. 7,265,190, one or more species are used. For ease of handling and the standpoint of physical properties of a water-absorbing resin or a particulate water-absorbing agent to be obtained, preferably peroxides or azo compounds, more preferably peroxides, and even more preferably persulfates are used.

In the embodiment of the present invention, an amount of the foregoing polymerization initiators used is preferably 0.001 to 1 mol%, more preferably 0.001 to 0.5 mol% per a monomer. An amount of the reducing agent used is preferably 0.0001 to 0.02 mol% per a monomer.

Instead of the foregoing polymerization initiators, polymerization reaction can be carried out by irradiating active energy beams such as radial rays, electron rays or ultraviolet rays, and it is also possible to use said active energy beams in combination with the polymerization initiators.

(Polymerization method)

Methods of polymerization for the embodiment of the present invention are, from the viewpoint of water absorbency and manageability of polymerization control and the like, preferably a gas-phase spray droplets polymerization, an aqueous solution polymerization or a reverse phase suspension polymerization in a hydrophobic solvent, more preferably an aqueous solution polymerization or reverse phase suspension polymerization, even more preferably an aqueous solution polymerization, and yet even more preferably a continuous aqueous solution polymerization. A polymerization may be performed in a single stage or a multi-stage fashion with two or more stages, and especially for a reverse phase suspension polymerization, a multi-stage polymerization is favorable from the standpoint of particle diameter control, granulation (particle size enlargement caused by aggregation of spherical particles), or a removal of the heat of polymerization.

Preferred embodiments for the foregoing continuous aqueous solution polymerization include a continuous polymerization involving a kneader (disclosed in the U.S. Patent No. 6987151 or No. 6710141) and a continuous polymerization involving a belt (disclosed in the U.S. Patent No. 4893999 and No. 6241928, or the U.S. Patent Application Publication No. 2005/215734). The continuous aqueous solution polymerization enables a production of a water-absorbing resin or a particulate water-absorbing agent with high efficiency.

Further, a preferred example of the foregoing continuous aqueous solution polymerization includes a polymerization with a high initial temperature and a polymerization in high concentrations. The "polymerization with a high initial temperature" is a method of polymerization, in which a temperature of the aqueous monomer solution is preferably 30°C or higher, more preferably 35°C or higher, even more preferably 40°C or higher, and yet even more preferably 50°C or higher (the upper limit is a boiling point), and the "polymerization in high concentrations" is a method of polymerization where a polymerization occurs with a monomer concentration of preferably 30% by weight or more, more preferably 35% by weight or more, even more preferably 40% by weight or more, and yet even more preferably 45% by weight or more (the upper limit is a saturation concentration). Those polymerization methods can be used in combination.

Further, the aforementioned polymerization method can allow a solids content concentration to increase during polymerization. In such a case, an "increase in solids content" identified by Formula (4) and Formula (5) is used as an index for evaluation. For the embodiment of the present invention, the increase in solids content is preferably 1% by weight or more, and more preferably 2% by weight or more.

[Mathematical. 4]

$$\text{Increase in solids content (\% by weight)} = (\text{Solids content concentration in hydrogel after polymerization}) - (\text{Solids content concentration in aqueous monomer solution}) \dots (4)$$

Meanwhile, in Formula (4), the "Solids content concentration in aqueous monomer solution" indicates a value obtained by Formula (5), meaning content percentage (% by weight) for the components which become a solid after polymerization.

[Mathematical 5]

$$\text{Solids content concentration in aqueous monomer solution (\% by weight)} = \text{Weight of (monomer component + graft component + water-absorbing resin + other solids content)}/(\text{Weight of components in polymer system}) \times 100 \dots (5)$$

In Formula (5), the "weight of components in polymer system" indicates a total weight of an aqueous monomer solution, a graft component, a water-absorbing resin, and other solids content (for example, water-insoluble fine particles and the like), and a hydrophobic solvent for a reverse phase suspension polymerization is not included therein.

In the embodiment of the present invention, polymerization can be carried out in air atmosphere, although, from the viewpoint of color of a water-absorbing resin or a particulate water-absorbing agent to be obtained, it is carried out preferably under inert gas atmosphere such as nitrogen or argon. In this case, an oxygen concentration is controlled to be preferably 1% by volume or less. Further, as to oxygen dissolved in monomers or an aqueous monomer solution, it is preferable to substitute fully using an inert gas (for instance, the dissolved oxygen to be less than 1 (mg/l)).

Furthermore, in said aqueous monomer solution, bubbles (especially said inert gases) and various foaming agents (especially carbonates) can be dispersed and polymerized, conducting thereby foaming polymerization.

(2-3) Gel-crushing step

This is a step for gel-crushing a hydrogel obtained from the foregoing polymerization step by a gel crusher such as a kneader, a meat chopper or a cutter mill and the like to obtain a hydrogel in a particle shape (hereinafter referred to as a "particulate hydrogel"). If said polymerization step is kneader polymerization, a polymerization step and a gel-crushing step are conducted simultaneously.

As for a gel-crushing step for the embodiment of the present invention, a gel crushing method disclosed in the International Publication No. 2011/126079 is preferably applied.

(2-4) Drying step

This is a step for drying a hydrogel and/or a particulate hydrogel, which is obtained through said polymerization step and/or the gel-crushing step, to a desirable level of resin solids content so as to obtain a dry polymer.

The resin solids content is a value calculated based on the amount lost from drying (a change in weight when a 1g sample is heated at 180°C for 3 hours) and is preferably 80% by weight or more, more preferably 85 to 99% by weight, even more preferably 90 to 98% by weight, and yet even more preferably 92 to 97% by weight.

As to a drying method for the embodiment of the present invention, as long as the hydrogel and/or the particulate hydrogel is dried until the aforementioned resin solids content is obtained, one or more can be appropriately selected from the drying methods including: heat drying, hot air drying, reduced-pressure drying, fluid bed drying, infrared drying, microwave drying, drum dryer drying, azeotropic dehydration drying using a hydrophobic organic solvent or drying in high humidity with high-temperature water vapor and the like.

Amongst the various drying methods described above, the hot air drying is preferable from the viewpoint of drying efficiency. Band drying, where hot air drying is performed on a belt, can be more preferable. A temperature of the hot air is, from the viewpoint of color or water-absorbing properties of a water-absorbing resin or a particulate water-absorbing agent to be obtained, preferably 100 to 300°C, and more preferably 120 to 220°C.

When performing band drying for the embodiment of the present invention, band drying conditions described in the International Publication Nos. 2006/100300, 2011/025012, 2011/025013, 2011/111657 and or the like are preferably applied.

(2-5) Crushing step and classification step

This is a step for crushing a dry polymer obtained from the foregoing drying step (crushing step) and making adjustments to the particle size to fit within a predetermined range (classifying step), to obtain water-absorbing resin powder (for simplicity, the powder-form water-absorbing resin prior to performing surface crosslinking is referred to as "water-absorbing resin powder").

Types of apparatus used in said crushing step include, for example: a high-speed rotary crusher such as a roll mill, a hammer mill, a screw mill, a pin mill and the like; a vibration mill; a knuckle-type crusher; a cylindrical mixer; and the like, and they can be combined if needed.

As a method for adjusting the particle size in the classification step, for instance, sieve classification which is carried out using a JIS(Japanese Industrial Standard) standardized sieve (JIS Z8801-1 (2000)) and air stream classification are included. Particle size adjustment for the water-absorbing resin can be appropriately conducted not only at the crushing step or at the classification step, but also at the polymerization step (especially reverse phase suspension polymerization or liquid drop spray polymerization) and other steps (for example, granulating step).

To solve the problems described in the embodiment of the present invention, and also to produce the particulate water-absorbing agent according to the embodiment of the present invention more efficiently, the particle size of the water-absorbing resin powder obtained in this step should be controlled desirably to be within the range described below.

Namely, the weight average particle diameter (D50) is controlled to be: preferably 200 to 600 $\mu$m, more preferably 200 to 550 $\mu$m, and even more preferably 250 to 500 $\mu$m.

Furthermore, as a ratio of particles having a particle diameter of less than 150 $\mu$m (fine particles) to particles having a particle diameter of 850 $\mu$m or more (coarse particles), it is controlled to be: preferably 5% by weight or less, more preferably 3% by weight or less, and even more preferably 1% by weight or less (the lower limit is 0% by weight). Meanwhile, the ratio of the particles having a particle diameter of less than 850 $\mu$m is preferably 90 to 100% by weight, and more preferably 95 to 100% by weight per relative to the entire particles.

Furthermore, the weight ratio between the particles having a particle diameter of less than 300 $\mu$m and particles having a particle diameter of 300 $\mu$m or more but less than 850 $\mu$m is preferably 70:30 to 10:90, more preferably 60:40 to 15:85, even more preferably 50:50 to 20:80, and particularly preferably 40:60 to 25:75. Meanwhile, the "particles having a particle diameter of less than 300 $\mu$m" indicates the water-absorbing resin powder which passes through a JIS standardized sieve with an opening size of 300 $\mu$m. The "particles having particle diameter of 300 $\mu$m or more but less than 850 $\mu$m" indicates the water-absorbing resin powder, which is sifted through a JIS standardized sieve with an opening size of 850 $\mu$m but remains over a JIS standardized sieve with an opening size of 300 $\mu$m.

Logarithmic standard deviation ($\sigma\zeta$) of particle size distribution is controlled such that it is preferably 0.20 to 0.50, more preferably 0.25 to 0.40, and even more preferably 0.27 to 0.35.

Each of the particle size described above is measured using a JIS standardized sieve in accordance with the measuring methods disclosed in the U.S. Patent No. 7,638,570 or EDANA ERT420.2-02.

Furthermore, the aforementioned particle size is also applied for a water-absorbing resin after surface crosslinking (hereinafter referred to as "water-absorbing resin particles" for simplicity) or a water-absorbing resin to which a chelating agent has been added (hereinafter, it might be referred to as "water-absorbing resin granulate" for simplicity).

Therefore, it is preferable to carry out surface crosslinking or granulation process so that the above described particle size range is maintained. Meanwhile, the particles size of a particulate water-absorbing agent as a final product is described in section (3-7).

(2-6) Surface crosslinking step

This is a step for forming a portion having a higher crosslinking density on a surface layer of the water-absorbing resin powder (a part within a few tens of micrometers from the surface of water-absorbing resin powder) which is obtained through the above described steps, and include following steps (2-6-1) to (2-6-3).
According to this surface crosslinking step, a water-absorbing resin (water-absorbing resin particles), of which surface is crosslinked, is obtained through radical crosslinking, surface polymerization, or crosslinking reaction with the surface crosslinking agent on the surface layer of the water-absorbing resin powder.

(2-6-1) Mixing step

(Surface crosslinking agent)

For instance, the surface crosslinking agent used in the embodiment of the present invention includes, but not limited to, various organic or inorganic surface crosslinking agents. In particular, from the viewpoint of the physical properties of a water-absorbing resin or a particulate water-absorbing agent or the manageability of a surface crosslinking agent, an organic surface crosslinking agent capable of forming a covalent bond upon reaction with carboxyl group is preferable.
Specifically, one or more types of the surface crosslinking agent described in the 9th to 10th columns of the U.S. Patent No. 7,183,456 are used. In that case, a total amount of the surface crosslinking agent used is, relative to 100 parts by weight of water-absorbing resin powder, preferably 0.01 to 10 parts by weight, and more preferably 0.01 to 5 parts by weight.
Furthermore, it is preferable to use water at the time of adding a surface crosslinking agent to water-absorbing resin powder. It is more preferable to add in an aqueous solution. In that case, an amount of water used is, relative to 100 parts by weight of water-absorbing resin powder, preferably 0.1 to 20 parts by weight, and more preferably 0.5 to 10 parts by weight.
Furthermore, a hydrophilic organic solvent is applied to the embodiment of the present invention, if necessary. In that case, an amount of the hydrophilic organic solvent used is, relative to 100 parts by weight of water-absorbing resin powder, preferably 10 parts by weight or less, and more preferably 5 parts by weight or less.
Furthermore, additives can be added during a re-moistening step described below by mixing with a surface crosslinking agent (aqueous solution) within a range of 5 parts by weight or less, or they can be added during a separate mixing step.

(Method for addition and mixing)

As to a method for adding and mixing the aforementioned surface crosslinking agent, although it is not limited, the surface crosslinking agent and, water, hydrophilic organic solvent or a combination thereof, as a solvent, are prepared in advance, and then those are added and combined by spraying or dropwise against the water-absorbing resin powder. Spraying followed by mixing is more preferable.
A mixing device used for mixing the surface crosslinking agent and the water-absorbing resin powder is, but not limited to, preferably a high-speed stirring mixer, and more preferably a continuous high-speed stirring type mixer.

(2-6-2) Heat treatment step

Heat treatment for the mixture of the aforementioned surface crosslinking agent and water-absorbing resin powder causes a surface crosslinking reaction, and therefore water-absorbing resin particles, with their surface being crosslinked, are obtained.
For heat treatment, a publicly known dryer is used, but preferably, it is a paddle dryer. A temperature for heating (temperature of heating medium) is preferably 80 to 250°C, more preferably 100 to 220°C, and even more preferably 160 to 200°C.

(2-6-3) Cooling step

For the purpose of terminating the aforementioned surface crosslinking reaction or proceeding to a next step or other purposes, if needed, a cooling process is performed after surface crosslinking .

For the cooling process, a device, which is the same type as the publicly known dryer used for the step of heat treatment, is applied. The cooling temperature (temperature of cooling medium) is preferably 0 to 90°C, more preferably 20 to 85°C, and even more preferably 40 to 80°C.

(2-7) Re-moistening step

This is a step for adding at least one type of additives, which is selected from the group consisting of: a polyvalent metal salt compound, a cationic polymer, a chelating agent, an inorganic reducing agent, and $\alpha$-hydroxycarboxylic compound, to the water-absorbing resin particles that are obtained in the above surface crosslinking step.

The additive is preferably added in the form of an aqueous solution or slurry. In this case, as the water-absorbing resin is re-swollen with water, this step is called "re-moistening step". Meanwhile, the aforementioned additive can be added and mixed simultaneously with the surface crosslinking agent solution described above.

(Polyvalent metal salt and/or cationic polymer)

In the embodiment of the present invention, from the viewpoint of enhancing a water absorption speed (Vortex), enhancing a liquid permeability (SFC), or fluidity during moisture absorption of a water-absorbing resin or a particulate water-absorbing agent to be obtained, a polyvalent metal salt and/or a cationic polymer is preferably added.

Specifically, the polyvalent metal salt and/or the cationic polymer and an amount used thereof that are described in "[7] Polyvalent metal salt and/or cationic polymer" of the International Publication No. 2011/040530 can also be applied to the present invention.

($\alpha$-Hydroxycarboxylic acid compound)

In the embodiment of the present invention, from the viewpoint of preventing coloration of a water-absorbing resin or a particulate water-absorbing agent to be obtained, an $\alpha$-hydroxycarboxylic acid compound can be additionally added. Meanwhile, the "$\alpha$-hydroxycarboxylic acid compound" indicates carboxylic acid or a salt thereof having hydroxyl group in the molecules, and it is hydroxycarboxylic acid which has a hydroxyl group at the $\alpha$ position.

As for the $\alpha$-hydroxycarboxylic acid compound, an $\alpha$-hydroxycarboxylic acid compound and an amount used thereof that are described in "[6] $\alpha$-Hydroxycarboxylic acid compound" of the International Publication No. 2011/040530 can also be applied to the present invention.

Meanwhile, a specific compound or an amount used of the chelating agent or inorganic reducing agent are described in sections (3-1) and (3-2) below.

(2-8) Step for adding other additives

In order to grant various functions to a water-absorbing resin, it is possible to add an additive other than the ones described above. Specifically, examples of such an additive encompass a surfactant, a compound having phosphorus atom, an oxidizing agent, an organic reducing agent, water-insoluble inorganic fine particle, organic powder such as metal soap, a water soluble deodorizing agent, an antibacterial agent, pulp, thermoplastic fiber and the like. Further, as a surfactant, the surfactant disclosed in the International Publication No. 2005/075070 can preferably be applied.

Furthermore, an amount of those additives used is determined as appropriately in accordance with the purpose for use and is not particularly limited. However, it is preferably 0 to 3% by weight, and more preferably 0 to 1% by weight relative to the water-absorbing resin.

Furthermore, from the viewpoint of damage resistance, the water-absorbing resin (in particular, particulate water-absorbing agent) preferably contains polyol. The polyol is more preferably ethylene glycol, diethylene glycol, polyethylene glycol, propylene glycol, glycerin, or pentaerythritol. More preferably, it is polyethylene glycol or propylene glycol. An amount of the polyol used is determined appropriately in accordance with the purpose of use and is not particularly limited. However, it is preferably 0.005 to 5% by weight, and more preferably 0.01 to 1% by weight relative to the water-absorbing resin.

Meanwhile, a specific compound or an amount of the water insoluble inorganic fine particles and water soluble deodorizing agent used are described in sections (3-1) and (3-2) below.

(2-9) Other steps

In addition to the above described steps, it is possible to have, if needed, steps such as a granulating step, a sizing step, a fine powder removing step, a fine powder recycling step, or the like. Moreover, it is possible to further include one or more of the steps such as a transporting step, a storing step, a packaging step, and a reserving step. As described herein, the sizing step includes a fine powder removing step after surface crosslinking step and a step of classifying and crushing when the water-absorbing resin is aggregated to yield a size larger than intended size. Furthermore, the fine powder recycling step includes a step for adding, during any step for producing a water-absorbing resin, fine powder itself or hydrogel prepared to have large size by fine powder granulating step. Meanwhile, the same applies to the step for producing the particulate water-absorbing agent according to the embodiment of the present invention.

Particulate water-absorbing agent and method for producing same

The particulate water-absorbing agent according to the embodiment of the present invention is characterized in that it contains a water-absorbing resin obtained by the aforementioned production method as an example and a chelating agent and satisfies the following requirements.

Namely, the particulate water-absorbing agent of the embodiment of the present invention contains a water-absorbing resin as a main component and a chelating agent, the water-absorbing resin containing particles with a particle diameter of less than 300 μm, as specified by a JIS standardized sieve, and particles with a particle diameter of 300 μm or more but less than 850 μm, as specified by a JIS standardized sieve, wherein an amount of the chelating agent included is 0.001 to 5 parts by weight per 100 parts by weight of a water-absorbing resin in solid and satisfies Formula (1):
[Mathematical 6]

$$1.2 < C1/C2 < 4.0 \ \dots \ (1)$$

where, in Formula (1), C1 indicates an amount (ppm) of the chelating agent present in the particles of a particle diameter of less than 300 μm, as specified by a JIS standardized sieve, amongst particle components for the particulate water-absorbing agent, and C2 indicates that an amount (ppm) of the chelating agent present in the particles of a particle diameter of 300 μm or more but less than 850 μm, as specified by a JIS standardized sieve, amongst particle components for the particulate water-absorbing agent.

Furthermore, the value for "C1/C2" is adjusted such that it is essentially higher than 1.2, preferably higher than 1.3, more preferably higher than 1.5, and even more preferably higher than 1.7. The effect of the embodiment of the present invention is exhibited more significantly as the value increases. Meanwhile, the upper limit is adjusted such that it is essentially lower than 4.0, preferably lower than 3.9, more preferably lower than 3.7, and even more preferably lower than 3.5. When the value is 4.0 or higher, the effect corresponding to the amount of chelating agent used is not obtained, and thus not economically favorable.

According to the embodiment of the present invention, the chelating agent is mainly localized on the surface of the water-absorbing resin particles, and the presence of the chelating agent can be confirmed by polishing the particle surface. Hereinbelow, the method for producing the particulate water-absorbing agent and the physical properties thereof according to the embodiment of the present invention are described.

(3-1) Chelating agent

The particulate water-absorbing agent according to the embodiment of the present invention essentially contains a chelating agent to overcome problems according to the embodiment of the present invention. From the viewpoint of the effect, the chelating agent may be preferably either a polymerized compound or a non-polymerized compound, and it is preferably a non-polymerized compound. Furthermore, it is desired to have a chelating agent having preferably an element other than carbon, hydrogen, or oxygen, and more preferably nitrogen or phosphorus contained in the molecule.

Meanwhile, the expression "an element other than carbon, hydrogen, or oxygen is contained in the molecule" means that that the neutralizing counter ion in an acidic group is excluded. For examples, in COONa, an element other than carbon, hydrogen, or oxygen is contained in the molecule of a chelating agent in addition to the neutralizing Na$^+$. Representative examples of a substituent group include an amino group and a sulfonic acid group.

Specific examples of the chelating agent used in the embodiment of the present invention include: amino polyvalent carboxylic acid, organic polyvalent phosphoric acid, inorganic polyvalent phosphoric acid, amino polyvalent phosphoric acid, and tropolone derivatives. At least one compound selected from a group consisting of those compounds is used in the embodiment of the present invention. Meanwhile, the term "polyvalent" means that a plurality of functional groups are present in one molecule. Preferably, there are 2 to 30, more preferably 3 to 20, and even more preferably 4 to 10 functional groups.

From the viewpoint of the influence on polymerization or properties of a particulate water-absorbing agent to be obtained, the chelating agent has a molecular weight preferably of 100 to 5000, and more preferably of 200 to 1000. When the molecular weight is more than 5000, the aqueous monomer solution may have increased viscosity depending on the time point for addition of the chelating agent, and in such case polymerization control or homogeneous mixing is difficult to achieve, and therefore undesirable.

Examples of the amino polyvalent carboxylic acid include: iminodiacetic acid, hydroxyethyl iminodiacetic acid, nitrilotriacetic acid, nitrilotripropionic acid, ethylenediamine tetraacetic acid, diethylenetriamine pentaacetic acid, triethylene tetramine hexaacetic acid, trans-1,2-diaminocyclohexane tetraacetic acid, N,N-bis(2-hydroxyethyl)glycine, diaminopropanol tetraacetic acid, ethylenediamine dipropionic acid, N-hydroxyethylenediamine triacetic acid, glycol ether diaminetetraacetic acid, diaminopropane tetraacetic acid, N,N'-bis(2-hydroxybenzil)ethylenediamine-N,N'-diacetic acid, 1,6-hexamethylendiamine-N,N,N',N'-tetraacetic acid, and a salt thereof.

Examples of the organic polyvalent phosphoric acid include: nitriloacetic acid-di(methylenephosphinic acid), nitrilodiacetic acid-(methylenephosphinic acid), nitriloacetic acid-β-propionic acid-methylenephosphonic acid, nitrilotris(methylenephosphonic acid), and 1-hydroxyethylidenediphosphonic acid.

Examples of the inorganic polyvalent phosphoric acid include pyrophosphoric acid, tripolyphosphoric acid, and a salt thereof.

Examples of the amino polyvalent phosphoric acid include: ethylenediamine-N,N'-di(methylenephosphinic acid), ethylenediaminetetra(methylenephosphinic acid), cyclohexanediaminetetra(methylenephosphonic acid), ethylenediamine-N,N'-diacetic acid-N,N'-di(methylenephosphonic acid), ethylenediamine-N,N'-di(methylenephosphonic acid), ethylenediaminetetra(methylenephosphonic acid), polymethylenediaminetetra(methylenephosphonic acid), diethylenetriaminepenta(methylenephosphonic acid), and a salt thereof.

Examples of the tropolone derivatives include tropolone, β-thujaplicin, and γ-thijaplicin.

Amongst them, a chelating agent selected from amino polyvalent carboxylic acid, organic polyvalent phosphoric acid, and amino polyvalent phosphoric acid is preferable. A chelating agent of amino polyvalent carboxylic acid having 3 or more carboxylic groups is more preferable. From the viewpoint of gel stability, diethylenetriamine pentaacetic acid, triethylene tetramine hexaacetic acid, trans-1,2-diaminocyclohexane tetraacetic acid, and a salt thereof are even more preferable.

In the embodiment of the present invention, the content (an amount used) of the chelating agent is, relative to 100 parts by weight of the solids content in water-absorbing resin, 0.001 to 5 parts by weight, preferably 0.02 to 1 parts by weight, more preferably 0.03 to 0.9 parts by weight, even more preferably 0.04 to 0.8 parts by weight, even sill more preferably 0.05 to 0.7 parts by weight, particularly preferably 0.06 to 0.6 parts by weight, and most preferably 0.07 to 0.5 parts by weight. When the content is more than 5 parts by weight, the effect corresponding to addition is not obtained so that it is not economically favorable and also problems like reduced absorption occur. On the other hand, when the content is less than 0.02 parts by weight, in particular less than 0.001 parts by weight, the gel stability or the effect of preventing coloration is not obtained at sufficient level.

In the embodiment of the present invention, the time point for adding the chelating agent is not particularly limited, and it can be added during any one or more steps of each step described for above [2] Method for producing polyacrylic acid (salt)-based water-absorbing resin. Furthermore, an amount used in each step corresponds to the content in a particulate water-absorbing agent which is substantially obtained.

Similar to the quantification of residual monomers or water soluble components, the chelating agent in the particulate water-absorbing agent can be suitably quantified by extracting the chelating agent in water or physiological saline and quantifying it using liquid chromatography or ion chromatography.

According to the embodiment of the present invention, because the chelating agent is preferably blended in surface of a water-absorbing resin, the chelating agent is preferably added during the surface crosslinking step or the re-moistening step after surface crosslinking step. Because deterioration of a water-absorbing resin progresses from resin surface, as the chelating agent is placed near the surface of a water-absorbing resin, the gel stability can be improved.

(3-2) Method for adding chelating agent

The method for producing a particulate water-absorbing agent according to the embodiment of the present invention is a method for producing a particulate water-absorbing agent containing a water-absorbing resin as a main component and a chelating agent, which comprises particles with a particle diameter, of less than 300 μm and particles with a particle diameter, as specified by a JIS standardized sieve, of 300 μm or more but less than 850 μm wherein a chelating agent content is 0.001 to 5 parts by weight relative to 100 parts by weight of the water-absorbing resin and satisfies Formula (1) .

[Mathematical 7]

$$1.2 < C1/C2 < 4.0 \ldots (1)$$

Meanwhile, in Formula (1), C1 indicates a chelating agent content (ppm) which is present in the particles with the particle diameter of less than 300 $\mu$m in which the particle diameter is specified by a JIS standardized sieve amongst the particles constituting the particulate water-absorbing agent and C2 indicates a chelating agent content (ppm) which is present in the particles with the particle diameter of 300 $\mu$m or more but less than 850 $\mu$m in which the particle diameter is specified by a JIS standardized sieve amongst the particles constituting the particulate water-absorbing agent.

More specifically, according to the production method of the following (a) to (d), in particular (a) or (b), a particulate water-absorbing agent satisfying Formula (1) can be obtained.

(a) Production method including a step of adding a chelating agent to a water-absorbing resin with crosslinked surface to obtain a granulate (particulated granulate) of a water-absorbing resin with increased weight average particle diameter (D50) 1.01 to 10 times relative to the weight average particle diameter before adding chelating agent), and a step of disintegrating the granulate (particulated granulate) of the water-absorbing resin to lower the weight average particle diameter (D50) by 5% or more (for example, the weight average particle diameter after disintegration is 0.1 to 0.95 times relative to the granulate).

(b) Production method including a step of classifying a surface crosslinked water-absorbing resin into several different particle sizes (for example, step of classifying into two particle sizes by using a JIS standardized sieve with opening size of 300 $\mu$m), a step of adding a chelating agent for each of the several different particle sizes (for example, a step of adding a larger amount of chelating agent to the particles with particle size of less than 300 $\mu$m compared to the particles with particle size of 300 $\mu$m or more, in particular, in the amount of 1.2 to 4.0 times), and a step of re-mixing several water-absorbing resins after addition of the chelating agent to obtain a particulate water-absorbing agent (for example, a step of re-mixing the particles which have been classified by using a JIS standardized sieve with opening size of 300 $\mu$m).

(c) Production method including a step of mixing and preparing a predetermined amount of particulate water-absorbing agent with different particle size distribution.

(d) Production method include a step of classifying a water-absorbing resin before surface crosslinking into several different particle sizes, a step of performing surface crosslinking and addition of a chelating agent for each of the different particle sizes, and a step of re-mixing several water-absorbing resins obtained after the surface crosslinking and addition of chelating agent to obtain a particulate water-absorbing agent.

Meanwhile, in order to achieve the subject of the embodiment of the present invention, the chelating agent is added for each different particle size of the water-absorbing resin, preferably before drying, and more preferably after classification, and even more preferably after surface crosslinking. It is also possible to have a step of adding a chelating agent to monomers during polymerization or a hydrogel forming crosslinked polymer before drying. The added amount of the chelating agent relative to monomers or a hydrogel forming crosslinked polymer is, relative to the whole amount added, preferably 0 to 50 mol%, more preferably 0 to 30 mol%, and even more preferably 0 to 10 mol%.

The chelating agent may be added to a water-absorbing resin without using a solvent. When the chelating agent is a solid, in particular, it may be powder mixing (dry blend). However, from the viewpoint of the fixing property on a surface of a water-absorbing resin, the chelating agent is preferably added as a solution or an aqueous dispersion. Examples of the solvent or dispersion medium which may be used therefor include water, a hydrophilic organic solvent, and a mixture solvent of water and a hydrophilic organic solvent.

Specific examples of the hydrophilic organic solvent include monovalent alcohols of C1 to C10, or C2 to C6 or so, polyols (for example, propylene glycol and glycerin), polyether polyols such as polyethylene glycols or porlypropylene glycols and an alkoxylated compound thereof (for example, poropoxyether of glycerin and methoxypolyethylene glycol). Amongst them, polyols, polyether polyols, or an alkoxylated compound thereof is preferable. From the viewpoint of controlling the dust property, polyols of C2 or C6 or so are particularly preferable.

When the chelating agent is added as a solution or an aqueous dispersion, it is added at, as a solution or an aqueous dispersion, preferably 0.1 to 25% by weight, more preferably 0.5 to 20% by weight, and even more preferably 1.0 to 10% by weight relative to the water-absorbing resin. Furthermore, it is sufficient that the concentration of the chelating agent in the solution or aqueous dispersion is 1 to 50% by weight or so. If necessary, a surfactant or the like can be used. The solvent can be dried, if necessary.

(3-3) Additives other than chelating agent

In addition to the above chelating agent, the particulate water-absorbing agent according to the embodiment of the present invention may be added with various additives for obtaining desired performances. Preferred examples of the additives include water insoluble inorganic fine particles, an inorganic reducing agent, a water soluble deodorizing agent, and polyvalent metal salts (with the proviso that, iron ions are excluded).

(Water insoluble inorganic fine particles)

When a water-absorbing agent having a water-absorbing resin as a main component is blended in an absorbent article like disposable diaper, fluidity of the water-absorbing agent is lowered in an environment with high humidity, thus making it difficult to have homogeneous mixing. Such difficulty is a cause of having particle segregation in water-absorbing resin which is admixed in an absorbent article.

Accordingly, it is important in the embodiment of the present invention to prevent blocking during moisture absorption, and it is desirable to add water insoluble inorganic particles to ensure the fluidity.

Specific examples of the water insoluble inorganic fine particles include metal oxide like silicon dioxide, titan oxide, zinc oxide, and aluminum oxide, calcium phosphate, silicic acid (salt) like natural zeolite and synthetic zeolite, kaolin, talc, clay, bentonite, and hydrotalcite. Amongst them, inorganic fine particles having average particle diameter of 200 $\mu$m or less, or 20 $\mu$m or less, in which the average particle diameter is measured by Coulter counter method, in particular silicon dioxide and silicic acid (salt) are more preferable.

The amount of the water insoluble inorganic fine particles added is, relative to the water-absorbing resin, preferably 0.05 to 1.0% by weight, more preferably 0.05 to 0.8% by weight, even more preferably 0.05 to 0.7% by weight, and particularly preferably 0.1 to 0.5% by weight. When the added amount is 0.05% by weight or more, a decrease in gel stability can be suppressed. On the other hand, when the added amount is 1.0% by weight or less, a decrease in the fluid retention capacity under pressure (AAP) of a particulate water-absorbing agent can be suppressed.

(Inorganic reducing agent)

Inorganic reducing agents mean a compound having reducing inorganic elements, which are added to prevent coloration or reduce residual monomers. Specific examples thereof include compounds having reducing sulfur atoms or reducing phosphorus atoms. In the embodiment of the present invention, it can be either an inorganic compound or an organic compound. As it can further enhance the gel stability and achieve the subject of the embodiment of the present invention, an inorganic reducing agent is preferable.

The inorganic reducing agent may be in acid form or salt form, but is preferably in salt form. Monovalent or polyvalent metal salts are more preferred, and monovalent metal salts are even more preferred. Amongst them, the oxygen-containing reducing inorganic compounds listed below as examples, that is, inorganic reducing agents in which sulfur or phosphorus is bonded to oxygen are preferred. The oxygen-containing reducing inorganic salts are more preferred.

Also, the inorganic reducing agent may also be an inorganic reducing agent formed from organic compounds having an alkyl group, a hydroxyalkyl group and the like, which carry reducing inorganic atoms, and preferably reducing sulfur atoms or phosphorus atoms.

Furthermore, as for the inorganic reducing agent carrying a reducing sulfur atom or a reducing phosphorus atom, examples include: sulfur compounds having valency of +4 (for example, sulfite, bisulfite, and pyrosulfite), sulfur compounds having valency of +3 (for example, dithionite), sulfur compounds having valency of +2 (for example, sulfoxylate), phosphorus compounds having valency of +4 (for example, hypophosphate), phosphorus compounds having valency of +3 (for example, phosphite and pyrophosphorous phosphate), and phosphorus compounds having valency of +1 (for example, hypophosphite). Meanwhile, sulfur compounds having valency of +6 (valency of positive number 6) and phosphorus compounds having valency of +5 (valency of positive number 5) are most stable, and each element having oxidation number equal to or lower than them has a reducing property. Furthermore, the reducing sulfur atoms or reducing phosphorus atoms may be replaced with an organic substance.

Foregoing inorganic reducing agents carrying a reducing sulfur atom include, but not limited to, for instance, inorganic compounds including: sulfites such as sodium sulfite, potassium sulfite, calcium sulfite, zinc sulfite, and ammonium sulfite; bisulfites such as sodium bisulfite, potassium bisulfite, calcium bisulfite, and ammonium bisulfite; pyrosulfites such as sodium pyrosulfite, potassium pyrosulfite, and ammonium pyrosulfite; dithionites such as sodium dithionite, potassium dithionite, ammonium dithionite, calcium dithionite, and zinc dithionite; trithionates such as potassium trithionate and sodium trithionate; tetrathionates such as potassium tetrathionate and sodium tetrathionate; thiosulfates such as sodium thiosulfate, potassium thiosulfate, and ammonium thiosulfate; and a water soluble organic compound such as 2-hydroxy-2-sulfinate acetic acid, sodium formaldehyde sulfoxylate, formamidine sulfinic acid and thioglycolic acid tris(2-carboxyethyl)phosphine hydrochloride (TCEP), and tributylphosphine (TBP) and the like. Amongst the water soluble organic compounds, preferred examples are 2-hydroxy-2-sulfinate acetic acid, 2-hydroxy-2-sulfonatoacetate and/or a salt thereof. Preferred salts include alkali metal and alkaline earth metal salts, and preferred examples include lithium salts, sodium salts, and potassium salts, while sodium salts are particularly preferred. 2-Hydroxy-2-sulfinate acetic acid (salt) may be used in combination with 2-hydroxy-2-sulfonatoacetic acid (salt), or in combination with the inorganic compounds that are described above.

The inorganic reducing agent carrying a reducing phosphorus atom includes, but not limited to, for instance, sodium hypophosphite.

In particular, preferred are sulfite, bisulfite, pyrosulfite, and dithionite. More preferred are sodium sulfite, sodium bisulfite, potassium pyrosulfite, and sodium dithionite.

An amount of the inorganic reducing agent added is, relative to the water-absorbing resin, preferably 0.01 to 1.0% by weight, more preferably 0.05 to 1.0% by weight, and even more preferably 0.05 to 0.5% by weight. When the added amount is 0.01% by weight or more, coloration with the lapse of time can be suppressed. Furthermore, when the added amount is 1.0% by weight or less, malodor can be suppressed, and in particular, malodor after absorbing an aqueous solution can be effectively suppressed.

(Water soluble deodorizing agent)

The water soluble deodorizing agent is added to provide the particulate water-absorbing agent with a deodorizing property. Meanwhile, the reason why a water soluble one is used as the deodorizing agent is that it can give an excellent deodorizing property to the particulate water-absorbing agent without deteriorating the water absorption performance. Herein, the expression "water soluble" means that not less than 0.1 g, preferably not less than 1 g, more preferably not less than 10 g is dissolved in 100 g of water at 25°C.

As for such water soluble deodorizing agent, various known ones such as extracts from leaves of Theaceae plants are usable. Example of the "Theaceae plants" include camellia, tea plant, sasanqua camellia, sakaki plant, and scarlet sakaki plant, and deodorizable components are extracted from their leaves with organic solvents, such as alcohols and ketones, or water, or their mixed solvents. Examples of the deodorizing components include flavonol, flavanols, organic polymers, and tannin.

An amount of the water soluble deodorizing agent added is, relative to the water-absorbing resin, preferably 0.0001 to 10% by weight. When the added amount is less than 0.0001% by weight, the deodorizing effect is not obtained. Meanwhile, when the added amount is more than 10% by weight, the effect is not obtained as much as the increased cost.

(Polyvalent metal salt)

The polyvalent metal salt (with the proviso that, iron ions are excluded) is added to improve the water absorbent speed or liquid permeability or to increase the fluidity during moisture absorption. Examples of the polyvalent metal salt include an organic acid salt or an inorganic acid salt which contains at least one metal (polyvalent metal cation) selected from typical metal or transition metal of Group 4 to Group 11. For example, an organic acid salt or an inorganic acid salt which contains at least one metal (polyvalent metal cation) selected from Mg, Ca, Ti, Zr, V, Cr, Mn, Co, Ni, Pd, Cu, Zn, Cd, and Al is preferable. Specific examples include aluminum sulfate, aluminum lactate, aluminum polychloride, magnesium sulfate, and zirconium sulfate.

(Other additives)

In addition to the aforementioned water insoluble inorganic fine particles, inorganic reducing agent, water soluble deodorizing agent, and polyvalent metal salt (with the proviso that, iron ions are excluded), an anti-bacterial agent, a fragrance, a foaming agent, a pigment, a dye, a plasticizer, an adhesive, a surfactant, a fertilizer, an oxidizing agent, salts, a bactericidal agent, a hydrophilic polymer like polyethylene glycol and polyethyleneimine, a hydrophobic polymer like paraffin, a thermoplastic resin like polyethylene and polypropylene, a thermosetting resin like polyester resin or urea resin, or the like can be suitably added to the particulate water-absorbing agent of the embodiment of the present invention to have desired functions.

Meanwhile, those additives may be added either simultaneously or separately with the chelating agent described above. The amount of the additives to be added is, relative to 100 parts by weight of the water-absorbing resin, preferably 30 parts by weight or less, more preferably 10 parts by weight or less, and even more preferably 1 part by weight or less.

(Method for adding additives)

The method for adding the water insoluble inorganic fine particles, inorganic reducing agent, water soluble deodorizing agent, or polyvalent metal salt to the water-absorbing resin is not particularly limited, and it can be performed either simultaneously or separately with the chelating agent described above. Furthermore, a so-called wet mixing method in which addition is performed after suspension in water or the like can be employed, or a dry blend method in which powders are admixed with each other can be employed.

Furthermore, each material described above can be added at any time. However, except the inorganic reducing agent, it can be preferably added after the drying step, more preferably before the surface crosslinking step or during the surface crosslinking step, and even more preferably after surface crosslinking step. The inorganic reducing agent is added preferably to a hydrogel forming crosslinked polymer before drying, more preferably after the drying step, even more preferably before the surface crosslinking step or during the surface crosslinking step, and particularly preferably after the surface crosslinking step.

(Addition for each different particle size)

In the embodiment of the present invention, the amount added of the additives other than the chelating agent, that is, the water insoluble inorganic fine particles, inorganic reducing agent, water soluble deodorizing agent, or polyvalent metal salt (with the proviso that, iron ions are excluded), is also different for each different particle size. In particular, it is preferable that they are added in a larger amount to the particles with small particle size.

Namely, amongst the particles constituting the particulate water-absorbing agent, when "B1" is the amount (ppm) of additives other than chelating agent which are present in particles with the particle diameter of less than 300 $\mu$m in which the particle diameter is specified by a JIS standardized sieve and "B2" indicates the amount (ppm) of additives other than chelating agent which are present in particles with the particle diameter of 300 $\mu$m or more but less than 850 $\mu$m in which the particle diameter is specified by a JIS standardized sieve, adjustment is made such that the value calculated as "B1/B2" is preferably more than 1.2, more preferably more than 1.3, even more preferably more than 1.5, and particularly preferably more than 1.7. The effect of the embodiment of the present invention is exhibited more significantly as the value increases. Meanwhile, the upper limit is adjusted such that it is essentially lower than 4.0, preferably lower than 3.9, more preferably lower than 3.7, and even more preferably lower than 3.5. When the value is 4.0 or higher, the effect corresponding to the amount of additives used other than chelating agent is not obtained, and thus not economically favorable.

(3-4) Amount of soluble contents

From the viewpoint of tackiness caused by eluted components during use of disposable diaper, the amount of soluble contents in the particulate water-absorbing agent of the embodiment of the present invention (soluble amount per hour specified by Examples that are described below) is controlled such that it is preferably 5 to 30% by weight, more preferably 5 to 25% by weight, and even more preferably 5 to 20% by weight in terms of a value with corrections for moisture content. The control method can be suitably performed by using an internal crosslinking agent or the like. Meanwhile, in order to obtain a particulate water-absorbing agent in which the amount of soluble contents is less than 5% by weight, a large amount of the internal crosslinking agent needs to be used. As such, the fluid retention capacity without pressure (CRC) is significantly lowered in addition to cost increase and an occurrence of residual crosslinking agent, and therefor undesirable.

(3-5) Amount of deteriorated solubles

The amount of deteriorated solubles of the particulate water-absorbing agent according to the embodiment of the present invention (the amount of deteriorated solubles per hour specified by Examples that are described below) is controlled such that it is preferably 0 to 40% by weight, more preferably 0 to 35% by weight, and even more preferably 0 to 31% by weight in terms of a value corrected for moisture content. When the amount of deteriorated solubles is more than 40% by weight, gel deterioration is caused by urine during use of disposable diaper, and it may cause leakage or reverse liquid flow.

Furthermore, according to the embodiment of the present invention, the difference in the amount of deteriorated solubles depending on particle size is small. As such, by combining the particulate water-absorbing agent of the embodiment of the present invention to an absorbent article like disposable diaper, only a small variation in the amount of deteriorated solubles is caused during actual use of disposable diaper.

The difference in the amount of deteriorated solubles (defined by (the amount of deteriorated solubles of particles with the particle diameter of less than 300 $\mu$m) - (the amount of deteriorated solubles of particles with the particle diameter of 300 $\mu$m or more but less than 850 $\mu$m)) is preferably 5% by weight or less, more preferably 3% by weight or less, even more preferably 2% by weight or less, and particularly preferably 1% by weight or less. The ratio between them (defined by (the amount of deteriorated solubles of particles with the particle diameter of less than 300 $\mu$m)/(the amount of deteriorated solubles of particles with particle diameter 300 $\mu$m or more but less than 850 $\mu$m)) is preferably 1.1 times or less, more preferably 1.05 or less, and even more preferably 1.02 times or less.

(3-6) Increased amount of deteriorated solubles

The increased amount of deteriorated solubles of the particulate water-absorbing agent according to the embodiment of the present invention (as specified by Examples that are described below is controlled such that it satisfies the following conditions. Namely, in the particles constituting the particulate water-absorbing agent, 1) the increased amount of deteriorated solubles of the particles with the particle diameter of less than 300 $\mu$m, in which the particle diameter is specified by a JIS standardized sieve, is 0 to 15% by mass, and 2) the increased amount of deteriorated solubles of the particles with the particle diameter of 300 $\mu$m or more but less than 850 $\mu$m, in which the particle diameter is specified by a JIS standardized sieve, is 0 to 20% by mass.

The increased amount of deteriorated solubles of the particles with the particle diameter of less than 300 $\mu$m is preferably 0 to 14% by weight, and more preferably 0 to 12% by weight. Furthermore, the increased amount of deteriorated solubles of the particles with the particle diameter of 300 $\mu$m or more but less than 850 $\mu$m is preferably 0 to 18% by weight, and more preferably 0 to 16% by weight. Meanwhile, those values include corrections for moisture content.

As the increased amount of deteriorated solubles is within the aforementioned range, the water absorption performance can be maintained as it is designed even in an environment in which the particulate water-absorbing agent can undergo deterioration. For example, even for a case where the particulate water-absorbing agent is used as a disposable diaper for a long period time, the water absorption performance of a disposable diaper is not lowered so that urine leakage or troubles like skin irritation can be reduced.

Meanwhile, if the increased amount of deteriorated solubles is not within the above range, the water absorption performance is lowered over the time in an environment in which the particulate water-absorbing agent can undergo deterioration. For examples, when the particulate water-absorbing agent is used as a disposable diaper for a long period time, the water absorption performance of a disposable diaper is lowered so that urine leakage or troubles like skin irritation may increase.

(3-7) Particle size

The particle size (specified by JIS Z8801-01(2000)) of the particulate water-absorbing agent according to the embodiment of the present invention is controlled to the following range in order to achieve the subject of the embodiment of the present invention more easily and to produce the particulate water-absorbing agent more efficiently.

Namely, the weight average particle diameter (D50) of the particulate water-absorbing agent is controlled preferably to 200 to 600 $\mu$m, more preferably to 200 to 550 $\mu$m, and even more preferably to 250 to 500 $\mu$m.

Furthermore, the ratio of particles (fine particles) having particle diameter of less than 150 $\mu$m in the particulate water-absorbing agent is controlled such that it is preferably 5% by weight or less, more preferably 3% by weight or less, and even more preferably 1% by weight or less (the lower limit is 0% by weight). Meanwhile, the ratio of the particles with the particle diameter of less than 850 $\mu$m is preferably 90 to 100% by weight, and more preferably 95 to 100% by weight.

Meanwhile, the weight ratio between the particles with the particle diameter of less than 300 $\mu$m and the particles with the particle diameter of 300 $\mu$m or more but less than 850 $\mu$m in the particulate water-absorbing agent is preferably 70 : 30 to 10 : 90, more preferably 60 : 40 to 15 : 85, even more preferably 50 : 50 to 20 : 80, and particularly preferably 40 : 60 to 25 : 75. Meanwhile, the "particles with the particle diameter of less than 300 $\mu$m" indicate a particulate water-absorbing resin which passes through a JIS standardized sieve with opening size of 300 $\mu$m and the "particles with the particle diameter of 300 $\mu$m or more but less than 850 $\mu$m" indicate a particulate water-absorbing resin which passes through a JIS standardized sieve with opening size of 850 $\mu$m but remains on a JIS standardized sieve with opening size of 300 $\mu$m.

From the above, according to the preferred embodiment of the embodiment of the present invention, the ratio of the particles with the particle diameter of 150 $\mu$m or more but less than 850 $\mu$m, in which the particle diameter is specified by a JIS standardized sieve, is preferably 90% by weight or more in the particulate water-absorbing agent. Furthermore, the weight ratio between the particles with the particle diameter of less than 300 $\mu$m and the particles with the particle diameter of 300 $\mu$m or more but less than 850 $\mu$m, in which the particle diameter is specified by a JIS standardized sieve, is preferably 70 : 30 to 10 : 90.

The ratio of the particles with the particle diameter of 150 $\mu$m or more but less than 850 $\mu$m, in which the particle diameter is specified by a JIS standardized sieve, is more preferably 95% by weight or more, and even more preferably 98% by weight or more.

The arithmetic standard deviation of particle size distribution ($\sigma\zeta$) of the particulate water-absorbing agent is controlled such that it is preferably 0.20 to 0.50, more preferably 0.25 to 0.40, and even more preferably 0.27 to 0.35.

(3-8) Fluid retention capacity without pressure (CRC) (ERT441.2-02)

The fluid retention capacity without pressure (CRC) of the particulate water-absorbing agent according to the embodiment of the present invention is controlled such that, in terms of a value corrected for moisture content, 25 (g/g) or more, more preferably 30 (g/g) or more, and even more preferably 33 (g/g) or more for physiological saline. Furthermore, from the viewpoint of balance with other properties (for example, the fluid retention capacity under pressure (AAP)), the upper limit is 60 (g/g) or less, more preferably 50 (g/g) or less, and even more preferably 45 (g/g) or less. The fluid retention capacity without pressure (CRC) can be controlled based on the crosslinking density during polymerization or surface crosslinking.

Meanwhile, if the fluid retention capacity without pressure (CRC) is lower than 25 (g/g), only poor efficiency is yielded when used for a hygienic material like disposable diaper, and therefore undesirable.

(3-9) Fluid retention capacity under pressure (AAP) (ERT442.2-02)

The fluid retention capacity under pressure (AAP) of the particulate water-absorbing agent according to the embodiment of the present invention is controlled such that, in terms of a value corrected for moisture content, 15 (g/g) or more, more preferably 20 (g/g) or more, and even more preferably 23 (g/g) or more for physiological saline at a load of 2.06 kPa. Furthermore, from the viewpoint of balance with other properties (for example, the fluid retention capacity without pressure (CRC)), the upper limit is 40 (g/g) or less, more preferably 35 (g/g) or less, and even more preferably 33 (g/g) or less. The fluid retention capacity under pressure (AAP) can be controlled based on surface crosslinking.

Meanwhile, when the fluid retention capacity under pressure (AAP) is less than 15 (g/g) and it is used for an absorbent body of a hygienic material like disposable diaper, an absorbent body having low returning amount of liquid (referred to as "Re-Wet") is not obtained when pressure is applied to an absorbent body, and thus not desirable.

Taken together, according to a preferred embodiment of the embodiment of the present invention, the fluid retention capacity without pressure (CRC) of the particulate water-absorbing agent is 30 (g/g) or more after corrected for moisture content against physiological saline, and the fluid retention capacity under pressure (AAP) at the pressure of 2.06 kPa is 20 (g/g) or more after corrections for moisture content against physiological saline.

(3-10) Moisture content

A moisture content in the particulate water-absorbing agent according to the embodiment of the present invention (specified by the loss after drying for 3 hours at 180°C) is controlled such that it is preferably 0.5 to 15% by weight, more preferably 1.0 to 10% by weight from the viewpoint of water absorbent speed or impact resistance. The moisture content is adjusted to a preferred range during the drying step or re-moistening step described above.

(3-11) Amount of Fe (iron ions)

An amount of Fe in the particulate water-absorbing agent according to the embodiment of the present invention is, from the viewpoint of preventing coloration of a particulate water-absorbing agent, preferably 2 ppm or less, more preferably 1.5 ppm or less, even more preferably 1 ppm or less, and particularly preferably 0.5 ppm or less. Furthermore, the lower limit is, from the viewpoint of the cost of purification of base (in particular, caustic soda), preferably 0.001 ppm or more, and more preferably 0.01 ppm or more. Meanwhile, control of the Fe amount can be achieved by suitably purifying the materials of a water-absorbing resin, in particular, the base used for neutralization, for example, by removing Fe present in NaOH or NazCOs.

The amount of Fe present in a particulate water-absorbing agent or a base can be quantified by ICP emission spectrophotometric analysis specified in JIS K1200-6. With regard to details of the quantification method, reference can be made to International Publication No. 2008/090961.

Meanwhile, when Fe is contained at 5 ppm in caustic soda (molecular weight: 40), it may be contained at 1.7 ppm (= $5 \times (40 \times 0.75/88.5)$) in sodium acrylate with neutralization rate of 75 mol% (average molecular weight of 88.5).

(3-12) Initial color

The particulate water-absorbing agent according to the embodiment of the present invention is used for absorbent articles like disposable diaper, and it is required to be present as white powder. Thus, the initial color of the particulate water-absorbing agent has, based on Hunter's Lab color system using spectrophotometric colorimetric system, an L-value (lightness) of preferably 88 or more, more preferably 89 or more, and even more preferably 90 or more. Meanwhile, the upper limit of the L-value is 100, and no problem occurs in absorbent articles like disposable diaper due to color, provided that the L-value is 88 or more. Moreover, an a-value is in a range of preferably -3 to 3, more preferably -2 to 2, still more preferably -1 to 1. Moreover, a b-value is in a range of preferably 0 to 12, more preferably 0 to 10, still more preferably 0 to 9. Note that, as the L-value approaches 100, whiteness is increased, and as the a-value and the b-value approach 0, the color fades and becomes substantially white.

(3-13) Color with the lapse of time

The particulate water-absorbing agent according to the embodiment of the present invention is used for absorbent articles like disposable diaper, and it is required to maintain a clean white state even when it is stored for a long period of time under high humidity and high temperature conditions. Thus, the color with the lapse of time of the particulate water-absorbing agent of the embodiment of the present invention has, based on the Hunter's Lab color system, an L-value (Lightness) of preferably 80 or more, more preferably 81 or more, still more preferably 82 or more, particularly preferably 83 or more. Meanwhile, the upper limit is 100, and no problem occurs due to color even after long-term storage under high humidity and high temperature conditions, provided that the L-value is 80 or more. Moreover, an a-value is in a range of preferably -3 to 3, more preferably -2 to 2, still more preferably -1 to 1. Moreover, a b-value is in a range of preferably 0 to 15, more preferably 0 to 12, still more preferably 0 to 10. Note that, as the L-value approaches 100, whiteness is increased, and as the a-value and the b-value approach 0, the color fades and becomes substantially white.

(3-14) Blocking ratio after moisture absorption

The particulate water-absorbing agent according to the embodiment of the present invention has a blocking ratio after moisture absorption of preferably 30% by weight or less, more preferably 20% by weight or less, even more preferably 10% by weight or less, and particularly preferably 5% by weight or less. The lower limit is preferably 0% by weight, but it can be 0.1% by weight or so. As the blocking ratio after moisture absorption is within the aforementioned range, excellent powder handleability is obtained, and therefore desirable. Meanwhile, when the blocking ratio after moisture absorption is more than 30% by weight, the particulate water-absorbing agent exhibits poor fluidity during production of absorbent articles like disposable diaper, and thus problems like difficult production of disposable diaper or the like may be caused.

(3-15) SFC (Saline flow conductivity)

The SFC (saline flow conductivity) of the particulate water-absorbing agent according to the embodiment of the present invention is preferably 1 ($\times$ $10^{-7} \cdot cm^3 \cdot s \cdot g^{-1}$) or greater, and more preferably 10 ($\times$ $10^{-7} \cdot cm^3 \cdot s \cdot g^{-1}$) or greater. The upper limit is 1,000 ($\times$ $10^{-7} \cdot cm^3 \cdot s \cdot g^{-1}$) or less. When the SFC is lower than 1 ($\times$ $10^{-7} \cdot cm^3 \cdot s \cdot g^{-1}$), permeability of liquid including body fluid like urine or blood is low, and thus it is not suitable for an absorbent body of absorbent articles like disposable diaper. On the other hand, when the SFC exceeds 1,000 ($\times$ $10^{7} \cdot cm^3 \cdot s \cdot g^{-1}$) or greater, liquid including body fluid like urine or blood is not sufficiently absorbed and liquid leakage may occur, and thus it is not suitable for an absorbent body of a hygiene product like disposable diaper. Meanwhile, SFC can be controlled based on particle size, surface crosslinking agent, polyvalent metal salt, cationic polymer, or the like.

Use of particulate water-absorbing agent

The particulate water-absorbing agent according to the embodiment of the present invention can be used for various applications, however, as it is stable due to a small variation in properties, it is used for absorbent articles, in particular absorbent articles like disposable diaper, sanitary napkin, and incontinence pad. In that case, the particulate water-absorbing agent is molded into sheet-like form by preparing it as a composite with hydrophilic fiber or the like. Meanwhile, if no hydrophilic fiber is used, the particulate water-absorbing agent is fixed in paper, nonwoven fabric or the like to give absorbent articles.

The amount of the particulate water-absorbing agent (core concentration) in the absorbent articles is preferably 10 to 100% by weight, more preferably 30 to 100% by weight, and even more preferably 50 to 100% by weight. Furthermore, the amount of the particulate water-absorbing agent used is preferably 1 to 30 g, more preferably 5 to 20 g, and even more preferably 10 to 20 g per absorbent article (for example, per disposable diaper). Furthermore, it is preferable that the absorbent article is adjusted to have density in the range of 0.06 to 0.5 (g/cm$^3$) and grammage in the range of 0.01 to 0.2 (g/cm$^2$).

Meanwhile, specific examples of the fiber substrate used for absorbent articles include hydrophilic fiber like crushed wood pulp, cotton linter, crosslinked cellulose fiber, rayon, cotton, wool, acetate, and vinylon. Those fiber substrates are preferably air-layered.

EXAMPLES

The embodiment of the present invention is more specifically described with the Examples given below. However, the embodiment of the present invention is not limited to the Examples and the examples derived from a proper combination of technical means disclosed in respective different Examples are also encompassed in the scope of the embodiment of the present invention.

Meanwhile, a power source of an electric apparatus (including measurement of properties of the water-absorbing agent) used in Production Examples, Examples, and Comparative Examples was 200 V or 100 V, unless otherwise noted. Moreover, the properties of the water-absorbing agent of the embodiment of the present invention were measured at a room temperature (in a range of 20°C to 25°C) and at a relative humidity of 50% RH, unless otherwise noted.

[Measurement properties of particulate water-absorbing agent]

The method for measuring properties of particulate water-absorbing agent, which is described below, can also be suitably applied to measurement of properties of a water-absorbing resin. In that case, the embodiment of the present invention is made by substituting the "particulate water-absorbing agent" with a "water-absorbing resin".

Furthermore, even for a particulate water-absorbing agent which has been produced with the same raw materials or production method, the moisture content may greatly vary depending on the storage period or environment for storage. Other properties are also affected depending on the moisture content, and there can be a case where precise evaluation cannot be made.

Accordingly, in the embodiment of the present invention, the properties that are affected by moisture content (the following (b), (c), and (e) to (h)) were evaluated after correcting measured value for moisture content. The value corrected for moisture content is obtained by dividing the measured value by (1-moisture content/100). Furthermore, it is expressed as a value relative to 100 parts by weight of solids content of a water-absorbing resin.

(a) Particle size (weight average particle diameter (D50), content for different particle sizes)

The particle size of the particulate water-absorbing agent according to the embodiment of the present invention (weight average particle diameter (D50), an amount for each different particle sizes) was measured by using a JIS standardized sieve based on the measurement method described in the U.S. Patent Application Publication No. 2006/204755.

Meanwhile, the amount for different particle sizes can be obtained by measuring weight of each of particles with the particle diameter of less than 300 $\mu$m and particles with the particle diameter of 300 $\mu$m or more but less than 850 $\mu$m.

(b) Fluid retention capacity without pressure (CRC)

The water absorption capacity without pressure (CRC) of the particulate water-absorbing agent according to the embodiment of the present invention was measured on the basis of ERT441.2-02 (2002).

Meanwhile, because the fluid retention capacity without pressure (CRC) is affected by moisture content, in the embodiment of the present invention the evaluation was made by using a value corrected for moisture content.

As a specific examples of moisture content correction, when moisture content is 5% by weight and measured value of fluid retention capacity without pressure (CRC) is 30 (g/g), the value corrected for moisture content is 31.6 (g/g) (= 30/ (1 - 5/100)).

(c) Fluid retention capacity under pressure (AAP)

The fluid retention capacity under pressure (AAP) of the particulate water-absorbing agent according to the embodiment of the present invention was measured on the basis of ERT442.2-02 (2002). The measurement was performed with a load of 2.06 kPa (0.3 psi).

Meanwhile, because the fluid retention capacity under pressure (AAP) is affected by moisture content, in the embodiment of the present invention the evaluation was made by using a value corrected for moisture content.

(d) Moisture content

Moisture content of the particulate water-absorbing agent according to the present embodiment of the present invention was measured on the basis of ERT430.2-02 (2002). Meanwhile, in the embodiment of the present invention, the measurement was made while the sample amount was changed to 1.0 g and a drying temperature was changed to 180°C, respectively.

(e) Amount of chelating agent

An amount of the chelating agent in the particulate water-absorbing agent of the embodiment of the present invention was measured according to the method described below.

1 g of the particulate water-absorbing agent was added to 100 g of physiological saline (0.9% by weight aqueous solution of sodium chloride) and, by stirring for 1 hour at room temperature, the chelating agent in physiological saline was extracted (revolution number for stirring: 500 $\pm$ 50 rpm). Meanwhile, in the embodiment of the present invention, the particulate water-absorbing agent is classified in advance by using a JIS standardized sieve with opening size of 300 $\mu$m, and for each of the product passed through the sieve (pass-product/particle diameter of less than 300 $\mu$m) and the product not passed through the sieve (on-product/particle diameter of 300 $\mu$m or more and less than 850 $\mu$m), measurement of the chelating agent content was carried out.

After that, the expanded gel was filtered by using a filter paper (manufactured by Toyo Roshi Kaisha Ltd., No. 2, retained particle size specified by JIS P 3801: 5 $\mu$m).

After that, the obtained filtrate was further subjected to filtration for pretreatment of HPLC sample (chromatodisc 25A/aqueous system type, manufactured by Kurabo Industries Ltd., pore size: 0.45 $\mu$m) to measure the chelating agent content in filtrate with the use of high-performance liquid chromatography (HPLC).

An amount of chelating agent in the particulate water-absorbing agent was obtained by taking into consideration of dilution rate of the particulate water-absorbing agent in physiological saline while using the calibration curve obtained by measuring a monomer standard solution with known concentration as an external standard.

Meanwhile, the conditions for measuring HPLC were suitably modified depending on the type of a chelating agent. Specifically, the quantification was carried out as follows: Measurement condition 1 for diethylene triamine pentaacetic acid (DTPA), Measurement condition 2 for triethylene tetraamine hexaacetic acid (TTHA), and Measurement condition 3 for ethylene diamine tetra(methylenephosphonic acid (EDTMP).

Measurement condition 1:

<<Eluent>>; Mixture solution of 0.3 ml of 0.4 mol/L aqueous alum solution, 450 ml of 0.1 N aqueous solution of sodium hydroxide, 3 ml of 0.4 mol/L aqueous solution of tetra n-butyl ammonium hydroxide, 3 ml of sulfuric acid, 1.5 ml of ethylene glycol, and 2550 ml of ion exchange water.

<<Column>>; LichroCART 250-4 Superspher 100 RP-18e (4 $\mu$m) (manufactured by Merck)

<<Column temperature>>; 23 $\pm$ 2°C

<<Flow rate>>; 1 ml/min

<<Detector>>; UV, wavelength of 258 nm

Measurement condition 2:

<<Eluent>>; 0.01 mol/L aqueous solution of sodium carbonate, 0.01 mol/L aqueous solution of sodium hydrogen carbonate, and 0.002 mol/L aqueous solution of tetra n-butyl ammonium hydroxide, each in the same amount, were admixed with one another followed by addition of 2 N hydrochloric acid to adjust pH to 7.5.

<<Column>>; Shodex Asahipak ODP-40 4D (Showa Denko K.K.)

<<Column temperature>>; 40°C

<<Column temperature>>; 0.7 ml/min

<<Detector>>; UV, wavelength of 254 nm

<<Preparation of sample solution>>; To a filtrate obtained by extracting the chelating agent from particulate water-absorbing agent, 0.01 mol/L aqueous solution of iron chloride (III) in an amount larger than the chelating agent content was added (for example, about 2 mL of 0.01 mol/L aqueous solution of iron chloride (III) can be added to 20 mL aqueous solution in which the chelating agent content is 100 ppm).

Measurement condition 3:

<<Eluent>>; 0.003 mol/L aqueous solution of sulfuric acid

<<Column>>; Shodex IC NI-424 (Showa Denko K.K.)

<<Column temperature>>; 40°C

<<Column temperature>>; 1 ml/min

<<Detector>>; RI

Since the chelating agent content is affected by the moisture content, the value of the embodiment of the present invention is a value corrected for moisture content, and it is converted to the value relative to 100 parts by weight of the solids content of a water-absorbing resin.

Meanwhile, when a chelating agent containing acidic group was used, calculation was made on the assumption that all of the contained acidic groups were neutralized with alkali. For example, in the case of diethylene triamine pentaacetic acid, it was calculated in terms of pentasodium salt.

(f) Amount of soluble contents

The amount of soluble contents in the particulate water-absorbing agent of the embodiment of the present invention was measured according to the following method.

First, the pH electrode used for measurement of the amount of soluble contents was calibrated by using a pH standard buffer solution (pH 4.0, pH 7.0, pH 10.0).

Next, 50 ml of previously prepared physiological saline (0.9% by weight aqueous solution of sodium chloride) was weighed in a 100 ml glass beaker, and under stirring using a stirrer chip (length: 30 mm), 0.1 mol/L aqueous solution of sodium hydroxide was added dropwise till to have pH 10. The dripping amount (Vab [ml]) of that case was obtained as blank. Subsequently, 0.1 mol/L aqueous hydrochloric acid solution was added dropwise thereto till to have pH 2.7. The dripping amount (Vbb [ml]) of that case was also obtained as blank.

Subsequently, to a polypropylene container provided with a cover (volume: 250 ml), 200 ml of previously prepared physiological saline was added followed by addition of 1.0 g of the particulate water-absorbing agent. Meanwhile, in the embodiment of the present invention, the particulate water-absorbing agent was classified in advance by using a JIS standardized sieve with opening size of 300 $\mu$m, and for each of the product passed through the sieve (pass-product/particle diameter of less than 300 $\mu$m) and the product not passed through the sieve (on-product/particle diameter of 300 $\mu$m or more and less than 850 $\mu$m), measurement of the amount of solubles was carried out.

Next, by using a stirrer chip (length of 30 mm $\times$ outer diameter of 8 mm), stirring was performed for 1 hour at 500 $\pm$ 50 rpm under room temperature to extract solubles. After that, filtration was performed by using a filter paper (manufactured by Toyo Roshi Kaisha Ltd., No. 2, retained particle size specified by JIS P 3801: 5 $\mu$m) to obtain a filtrate.

20 ml of the filtrate obtained from above operations (recorded as F [ml]) was weighed in a glass beaker with volume of 100 ml, and according to mess-up to 50 ml with physiological saline, a liquid for titration was obtained. Meanwhile, even when the filtrate is less than 20 ml, the amount (F [ml]) was recorded, and according to mess-up to 50 ml with physiological saline, a liquid tor titration was obtained.

Subsequently, by using a stirrer chip (length of 30 mm $\times$ outer diameter of 8 mm), the liquid for titration was stirred and 0.1 mol/L aqueous solution of sodium hydroxide was added dropwise thereto till to have pH of 10. The titration amount (Va [ml]) was obtained. Subsequently, 0.1 mol/L aqueous hydrochloric acid solution was added dropwise thereto till to have pH of 2.7 and the titration amount (Vb [ml]) was obtained.

According to the following formulae (6) to (11), the amount of soluble contents was obtained.
[Mathematical 8]

$$\text{Amount of soluble contents (\% by weight)} = \{(Wa + Wb)/m\} \times 100 \ \dots \ (6)$$

Meanwhile, in Formula (6), "Wa" means the relative weight of a unit having acidic groups in the solubles of the particulate water-absorbing agent, "Wb" means the relative weight of a unit having carboxylate groups, which are neutralized by a basic substance, in the solubles of the particulate water-absorbing agent, and they are obtained by the following formula. Furthermore, "m" represents the weight of a particulate water-absorbing agent.
[Mathematical 9]

$$Wa \ (g) = Na \times 72 \times 200/F \ \dots \ (7)$$

[Mathematical 10]

$$Wb \ (g) = Nb \times 94 \times 200/F \ \dots \ (8)$$

Meanwhile, "72" in Formula (7) indicates weight per mole of repeating unit in an acrylic acid polymer and "94" in Formula (8) indicates weight per mole of repeating unit in a sodium acrylate polymer. However, when monomers having acidic groups other than acrylic acid are co-polymerized or other salt such as a potassium salt or a lithium salt is used as an alkali metal salt instead of a sodium salt, those values are calculated with suitable modification to average weight of repeating units including the monomer.
Furthermore, "Na" in Formula (9) indicates a number of moles for acidic groups in the solubles that are contained in a liquid for titration (filtrate), and "Nb" in Formula (10) indicates a number of moles for carboxylate groups, which are neutralized by a basic substance, in the solubles that are contained in a liquid for titration (filtrate). They are obtained by the following formula.
[Mathematical 11]

$$Na \ (mole) = (Va - Vab)/1000 \times 0.1 \ \dots \ (9)$$

[Mathematical 12]

$$Nb \ (mole) = N_1 - Na \ \dots \ (10)$$

Meanwhile, "$N_1$" in Formula (10) indicates a total number of moles for the solubles that are contained in a liquid for titration (filtrate), and it is obtained by the following Formula (11).
[Mathematical 13]

$$N_1 \ (mole) = (Vb - Vbb)/1000 \times 0.1 \ \dots \ (11)$$

Meanwhile, because the amount of soluble contents is affected by moisture content, in the embodiment of the present invention the evaluation was made by using a value corrected for moisture content.

(g) Amount of deteriorated solubles

The amount of deteriorated solubles in the particulate water-absorbing agent of the embodiment of the present invention was measured according to the following method.
Namely, as a solution for deterioration test, to a previously prepared physiological saline, L-ascorbic acid was added to make a 0.1% by weight of such a solution. Specifically, 1.0 g of L-ascorbic acid was added and then dissolved in 999.0 g of physiological saline.
Next, to a polypropylene container provided with a cover (volume: 250 ml), 200 ml of the above deterioration test solution was added followed by addition of 1.0 g of a particulate water-absorbing agent to form an expanded gel. Specifically, the container was sealed with a cover, and the expanded gel was allowed to stand for 2 hours in an atmosphere of 60°C. Thereafter, a stirrer chip (length of 30 mm × outer diameter of 8 mm) was added thereto, and stirring was performed for 1 hour in the same manner as above (amount of soluble contents) to extract the deteriorated solubles. After that, by performing the same measurement as the method for measuring the amount of soluble contents, the amount of deteriorated solubles was obtained (% by weight).

Meanwhile, because the amount of deteriorated solubles is affected by moisture content, in the embodiment of the present invention the evaluation was made by using a value corrected for moisture content.

(h) Increased amount of deteriorated solubles

The increased amount of deteriorated solubles in the particulate water-absorbing agent of the embodiment of the present invention is obtained by using Formula (2).
[Mathematical 14]

```
Increased amount of deteriorated solubles (% by weight) =
(Amount of 1 hour-solubles in deterioration test solution) -
(Amount of 1 hour-solubles in physiological saline) ... (2)
```

Meanwhile, the "increased amount of deteriorated solubles" is an index for evaluating the gel stability, and it means the amount of soluble contents which increases in accordance with deterioration of a particulate water-absorbing agent.

(i) Evaluation of coloration of particulate water-absorbing agent (Hunter Lab color values/L, a, b values)

Coloration of the particulate water-absorbing agent was evaluated by LabScan (registered trademark) XE manufactured by HunterLab. A reflection measurement was selected as a preset condition of measurement, and a container for powder and paste sample having internal diameter of 30 mm and height of 12 mm was used. Further, a standard round white plate No. 2 for powder · paste was used as a standard and 30$\phi$ light-transmitting pipe was used. About 5 g of a particulate water-absorbing agent was provided in the built-in sample container so as to occupy about 60% of the built-in sample container. Then, an L value (Lightness: lightness index) on a surface was measured by the spectrophotometric colorimeter at room temperature (20 to 25°C) and humidity of 50 RH %. This value indicates "lightness index before exposure". The higher index value indicates a color close to white.

Furthermore, by using the same device and the same measurement method, values of a and b (color) as an article color based on other scale can also be measured. Smaller values of a and b indicate low coloration, that is, a color substantially close to white color. The result of evaluating coloration of a particulate water-absorbing agent immediately after the production, or a particulate water-absorbing agent after storage for 1 year or less in an atmosphere at temperature of 30°C or lower and relative humidity of 50 RH % or less corresponds to the initial color.

Subsequently, after filling the container forpaste sample with about 5 g of a particulate water-absorbing agent, the container for paste sample filled with a particulate water-absorbing agent was exposed for 7 days in a constant temperature and constant humidity chamber (small-size environmental testing machine manufactured by Espec Corp.; Model SH-641) that had been adjusted to an atmosphere at a temperature of 70 ± 1°C and relative humidity of 65 ± 1 RH %. This exposure corresponds to the coloration acceleration test for 7 days. After the exposure, values of L, a, b on a surface were measured by using the aforementioned spectrophotometric colorimeter. The resulting measurement value was used as coloration over the time after acceleration test.

(j) Malodor test

One part by weight of a particulate water-absorbing agent was added to a 100 ml beaker. After adding 20 parts by weight of 0.9% by weight aqueous solution of sodium chloride, the beaker was sealed with a film and allowed to stand for 1 hour at 37°C. After that, the malodor sensory test was performed with 10 adult subjects. With regard to the evaluation method, when there is no unpleasant malodor so that the odor is determined to be acceptable for use in a hygienic material like diaper, it was evaluated as O. When there is unpleasant malodor so that the odor is determined to be unacceptable for use in a hygienic material like diaper, it was evaluated as ×. Particularly strong malodor was evaluated as ××.

(k) Blocking ratio after moisture absorption

In an aluminum cup having a bottom surface diameter of about 52 mm and a height of 22 mm, 2 g of a particulate water-absorbing agent was evenly scattered on the bottom and quickly transferred to a constant-temperature and constant-humidity incubator which has been adjusted in advance to 25°C and relative humidity of 90% (PLATIOOUS LUCIFER PL-2G manufactured by Tabai Espec Corp. Inc.), and it was allowed to stand for 60 minutes. After that, the water-absorbing agent after moisture absorption was transferred to a JIS standardized sieve with diameter of 7.5 cm and opening size of 2000 $\mu$m. At that time, when the water-absorbing agent after moisture absorption is strongly fixed onto the aluminum cup so that it cannot be transferred to a sieve, the water-absorbing agent which absorbs moisture and is in blocking state is carefully peeled off so as not to disrupt it as much as possible, and then transferred to a sieve. It was directly sieved for 8 seconds by using a vibration classifying device (IIDA SIEVE SHAKER, TYPE: ES-65, SER. No. 0501), and the weight of the water-absorbing agent remained on top of the sieve (Wo (g)) and the weight of the water-absorbing agent passed through the sieve (Wp (g)) were measured. According to the following calculation formula, the blocking ratio after moisture absorption (% by weight) was calculated. Smaller blocking ratio after moisture absorption indicates better moisture absorption fluidity and more improved powder handleability.

```
Blocking ratio after moisture absorption (% by weight) =
{Weight Wo (g)/(Weight Wo (g) + Weight Wp (g))} × 100
```

(I) SFC (Saline flow conductivity)

SFC (Saline flow conductivity) of the water-absorbing resin of the embodiment of the present invention was measured according to the measurement method described in the U.S. Patent No. 5,669,894.

[Preparation Example 1]

To 5500 g (monomer concentration of 38% by weight) of an aqueous sodium acrylate solution with neutralization rate of 75 mol% in which acrylic acid containing p-methoxyphenol at 70 ppm was neutralized with an aqueous solution of sodium hydroxide, 2.5 g of polyethylene glycol diacrylate (average molecular weight of 523) was dissolved as an internal crosslinking agent to give an aqueous monomer solution (1).

Next, to a reactor formed by adding a cover to a stainless steel kneader equipped with double-arm type jacket having two sigma-shaped blades (internal volume of 10 L), the above aqueous monomer solution (1) was introduced, and nitrogen gas was blown into the reactor to replace the inside of the system with nitrogen while maintaining the liquid temperature at 30°C.

Subsequently, while stirring the above aqueous monomer solution (1), 14.2 g of 20% by weight aqueous solution of sodium persulfate and 2.4 g of 1% by weight aqueous solution of L-ascorbic acid were added separately as a polymerization initiator. As a result, after about 1 minute, the polymerization reaction was initiated.

The polymerization was performed while crushing the produced hydrogel forming crosslinked polymer (hereinafter referred to as "hydrogel") (1), and the polymerization peak temperature of 90°C was exhibited after lapse of 15 minutes. After 60 minutes from the start of the polymerization, the hydrogel (1) was extracted. Meanwhile, the obtained hydrogel (1) was found to be finely crushed to have particle diameter of 1 to 4 mm.

Next, the finely crushed hydrogel (1) was spread on a wire mesh with opening size of 300 $\mu$m (50 mesh), and then dried with hot air at 180°C for 45 minutes. Subsequently, pulverization was carried out by using a roll mill, and classification was performed with JIS standardized sieves with opening size of 850 $\mu$m or 150 $\mu$m. According to this series of operations, the water-absorbing resin powder (1) in amorphous and crushed form of which particle diameter is 150 $\mu$m or more but less than 850 $\mu$m was obtained. The solids content in the water-absorbing resin powder (1) was 96% by weight.

Subsequently, by homogeneously mixing 100 parts by weight of the water-absorbing resin powder (1) with 3.52 parts by weight of a surface crosslinking agent solution containing 0.02 parts by weight of ethylene glycol diglycidyl ether, 0.5 parts by weight of propylene glycol, 2.7 parts by weight of ion exchange water, and 0.3 parts by weight of 1,4-butane diol and heating them for 40 minutes at 164°C, the water-absorbing resin particle (1) with crosslinked surface was obtained. A weight average particle diameter (D50) of the water-absorbing resin particle (1) was 446 $\mu$m.

[Example 1]

A mixed solution containing 0.1 part by weight of diethylene triamine pentaacetic acid·trisodium and 1 part by weight of ion exchange water was added dropwise to 100 parts by weight of the water-absorbing resin particle (1) which was obtained in Preparation Example 1 followed by homogenous mixing. After that, as a result of keeping it for 60 minutes in a hot air dryer at 60°C, a granulate product in which several particles are aggregated (hereinbelow, it is referred to as a "water-absorbing resin granulate" for the sake of convenience) was obtained. The weight average particle diameter (D50) of the water-absorbing resin granulate (1) of Example 1 was 477 $\mu$m (1.07 time the weight average particle diameter (D50) of the water-absorbing resin particle (1)).

Next, 30 g of the above water-absorbing resin granulate (1) and 10 g of glass beads with diameter of 6 mm were added to a glass container (diameter of 6 cm, and height of 11 cm), and the water-absorbing resin granulate (1) was disintegrated by using a paint shaker (product No. 488/Toyo Seiki Seisaku-Sho, Ltd.). Meanwhile, the disintegration was performed by shaking the paint shaker for 30 minutes at 800 (cycle/min) (CPM) . Details of the paint shaker are disclosed in JP No. 9-235378 A.

After the shaking, by removing the glass beads using a JIS standardized sieve with opening size of 2 mm, a particulate water-absorbing agent (1) was obtained. The weight average particle diameter (D50) of the particulate water-absorbing agent (1) was 430 $\mu$m (0.90 time the weight average particle diameter (D50) of the water-absorbing resin granulate (1), and 0.96 time the weight average particle diameter (D50) of the water-absorbing resin particle (1)). Meanwhile, properties like moisture content were shown in Table 1.

Furthermore, in the above particulate water-absorbing agent (1), the ratio of particles with particle diameter 150 $\mu$m or more but less than 850 $\mu$m, in which the particle diameter is specified by a JIS standardized sieve, was 99.3% by weight. The amount of deteriorated solubles for different particle sizes and particle distribution are shown in Table 1. Meanwhile, the amount of deteriorated solubles in the entire particulate water-absorbing agent (1) was 27.2% by weight.

[Example 2]

Except that the amount of diethylene triamine pentaacetic acid · trisodium that was used was changed to 0.05 parts by weight, the same operations as in Example 1 (granulation using 1.05 parts by weight of aqueous solution of chelating agent, disintegration using a paint shaker, and removal of glass beads) were performed to obtain a particulate water-absorbing agent (2).

The weight average particle diameter (D50) of the water-absorbing resin granulate (2) after the addition of a chelating agent was 480 $\mu$m (1.08 time the weight average particle diameter (D50) of the water-absorbing resin particle (1)), and the weight average particle diameter (D50) of the particulate water-absorbing agent (2) was 431 $\mu$m (0.90 time the weight average particle diameter (D50) of the water-absorbing resin granulate (2), and 0.97 time the weight average particle diameter (D50) of the water-absorbing resin particle (1)). Meanwhile, physical properties like moisture content are shown in Table 1.

Furthermore, in the above particulate water-absorbing agent (2), the ratio of particles with particle diameter 150 $\mu$m or more but less than 850 $\mu$m, in which the particle diameter is specified by a JIS standardized sieve, was 99.3% by weight. The amount of deteriorated solubles for different particle sizes and particle distribution are shown in Table 1. Meanwhile, the amount of deteriorated solubles in the entire particulate water-absorbing agent (2) was 27.5% by weight.

[Example 3]

Except that the amount of diethylene triamine pentaacetic acid·trisodium that was used was changed to 0.003 parts by weight, the same operations as in Example 1 (granulation using 1.003 parts by weight of aqueous solution of chelating agent, disintegration using a paint shaker, and removal of glass beads) were performed to obtain the particulate water-absorbing agent (3).

The weight average particle diameter (D50) of the water-absorbing resin granulate (3) after the addition of a chelating agent was 479 $\mu$m (1.07 time the weight average particle diameter (D50) of the water-absorbing resin particle (1)), and the weight average particle diameter (D50) of the particulate water-absorbing agent (3) was 426 $\mu$m (0.89 time the weight average particle diameter (D50) of the water-absorbing resin granulate (3), and 0.96 time the weight average particle diameter (D50) of the water-absorbing resin particle (1)). Meanwhile, physical properties like moisture content are shown in Table 1.

Furthermore, in the above particulate water-absorbing agent (3), the ratio of particles with particle diameter 150 $\mu$m or more but less than 850 $\mu$m, in which the particle diameter is specified by a JIS standardized sieve, was 99.3% by weight. The amount of deteriorated solubles for different particle sizes and particle distribution are shown in Table 1. Meanwhile, the amount of deteriorated solubles in the entire particulate water-absorbing agent (3) was 30.6% by weight.

[Comparative Example 1]

Except that the paint shaker of Example 3 is not used, the same operations as in Example 3 (granulation using 1.003 parts by weight of aqueous solution of chelating agent) were performed to obtain a comparative particulate water-absorbing agent (1).

The weight average particle diameter (D50) of the comparative water-absorbing resin granulate (1) after the addition of a chelating agent was 479 $\mu$m (1.07 time the weight average particle diameter (D50) of the water-absorbing resin particle (1)), and the weight average particle diameter (D50) of the comparative particulate water-absorbing agent (3) was also 479 μm (1.0 time the weight average particle diameter (D50) of the comparative water-absorbing resin granulate (1), and 1.07 times the weight average particle diameter (D50) of the water-absorbing resin particle (1)). Meanwhile, physical properties like moisture content are shown in Table 1.

Furthermore, in the above comparative particulate water-absorbing agent (1), the ratio of particles with particle diameter 150 μm or more but less than 850 μm, in which the particle diameter is specified by a JIS standardized sieve, was 99.3% by weight. The amount of deteriorated solubles for different particle sizes and particle distribution are shown in Table 1. Meanwhile, the amount of deteriorated solubles in the entire comparative particulate water-absorbing agent (1) was 30.9% by weight.

[Comparative Example 2]

Except that the amount of diethylene triamine pentaacetic acid·trisodium of Comparative Example 1 that was used was changed to 0.002 parts by weight, the same operations as in Comparative Example 1 (granulation using 1.002 parts by weight of aqueous solution of chelating agent) were performed to obtain a comparative particulate water-absorbing agent (2).

The weight average particle diameter (D50) of the comparative water-absorbing resin granulate (2) after the addition of a chelating agent was 479 μm (1.07 time the weight average particle diameter (D50) of the water-absorbing resin particle (1)), and the weight average particle diameter (D50) of the comparative particulate water-absorbing agent (2) was also 479 μm (1.0 time the weight average particle diameter (D50) of the comparative water-absorbing resin granulate (2), and 1.07 time the weight average particle diameter (D50) of the water-absorbing resin particle (1)). Meanwhile, physical properties like moisture content are shown in Table 1.

Furthermore, in the above comparative particulate water-absorbing agent (2), the ratio of particles with particle diameter 150 μm or more but less than 850 μm, in which the particle diameter is specified by a JIS standardized sieve, was 99.3% by weight. The amount of deteriorated solubles for different particle sizes and particle distribution are shown in Table 1. Meanwhile, the amount of deteriorated solubles in the entire comparative particulate water-absorbing agent (2) was 51.4% by weight.

[Example 4]

The water-absorbing resin particle (1) obtained from Preparation Example 1 was classified by using a JIS standardized sieve with opening size of 300 μm, and divided into a portion remained on the sieve (hereinbelow, described as "water-absorbing resin particle (1-on)") and a portion passed through the sieve (hereinbelow, described as "water-absorbing resin particle (1-pass)"). Meanwhile, the particle size distribution of the water-absorbing resin particle (1-on) was 300 μm or more but less than 850 μm and the particle size distribution of the water-absorbing resin particle (1-pass) was less than 300 μm.

Next, to 100 parts by weight of the water-absorbing resin particle (1-on), a mixed solution containing 0.01 parts by weight of diethylene triamine pentaacetic acid·trisodium and 1 part by weight of ion exchange water was added dropwise followed by homogenous mixing. To 100 parts by weight of the water-absorbing resin particle (1-pass), a mixed solution containing 0.025 parts by weight of diethylene triamine pentaacetic acid·trisodium and 1 part by weight of ion exchange water was added dropwise followed by homogenous mixing.

Subsequently, the mixture was allowed to stand for 60 minutes in a hot air dryer at 60°C followed by re-mixing. According to this series of the operations, a particulate water-absorbing agent (4) was obtained. Meanwhile, physical properties like moisture content are shown in Table 1.

Furthermore, in the above particulate water-absorbing agent (4), the ratio of particles with particle diameter 150 μm or more but less than 850 μm, in which the particle diameter is specified by a JIS standardized sieve, was 99.3% by weight. The amount of deteriorated solubles for different particle sizes and particle distribution are shown in Table 1. Meanwhile, the amount of deteriorated solubles in the particulate water-absorbing agent (4) was 28.8% by weight.

[Preparation Example 2]

To a polypropylene container covered with foamed styrol as an insulating material (volume: 1 L), 291 g of acrylic acid, 0.63 g of polyethylene glycol diacrylate (average molecular weight of 523) as an internal crosslinking agent, 1.80 g of 1.0% by weight aqueous solution of diethylene triamine pentaacetic acid·trisodium, and 3.60 g of 1.0% by weight acrylic acid solution of IRGACURE (registered trademark) 184 were added to prepare a mixture solution (a).

Furthermore, to a separate polypropylene container covered with foamed styrol as an insulating material (volume: 1 L), 247 g of 48.5% by weight aqueous solution of sodium hydroxide and 255 g of ion exchange water adjusted to 50°C were added to prepared a mixture solution (b).

While stirring the mixture solution (a) at 800 rpm using a magnetic stirrer (length of 5 cm), the mixture solution (b) was quickly added and mixed to obtain an aqueous monomer solution (2). According to neutralization heat and dissolution heat, the aqueous monomer solution (2) was heated to have liquid temperature of about 100°C. Meanwhile, the rate of neutralization of acrylic acid was 73.5 mol%. Furthermore, the amount of diethylene triamine pentaacetic acid·trisodium (chelating agent) was 0.005% by weight (50 ppm) relative to the monomer solids content.

Subsequently, by adding 1.8 g of 3% by weight aqueous solution of sodium persulfate as a polymerization initiator to the aqueous monomer solution (2), a reaction solution (2) was prepared.

The above reaction solution (2) was stirred for about 1 second, and immediately injected to a stainless vat type reaction apparatus (bottom surface; 340 × 340 mm, height; 25 mm, inner surface; coated with Teflon (registered trademark)) in an open air system. Furthermore, according to simultaneous irradiation of ultraviolet rays, the polymerization reaction was initiated about 10 seconds later. Within one minute thereafter, a peak temperature was shown.

When three minutes were passed after start of the polymerization reaction, the ultraviolet ray irradiation was terminated and the hydrogel forming crosslinked polymer (hereinafter referred to as "hydrogel") (2) was extracted. Meanwhile, the above series of the operations was performed in an open air system.

Subsequently, the hydrogel (2) was subjected to gel-crushing by using a meat chopper (MEAT-CHOPPER TYPE 12VR-400KSOX; die pore diameter: 6.4 mm, number of pores: 38, die thickness: 8 mm/ manufactured by Iizuka Kogyo Co., Ltd.) to obtain a finely crushed hydrogel (2).

Next, the finely crushed hydrogel (2) was spread on a wire mesh with opening size of 300 μm (50 mesh), and then dried with hot air at 180°C for 45 minutes. Subsequently, pulverization was carried out by using a roll mill, and classification was performed with JIS standardized sieves with opening size of 850 μm or 150 μm. According to this series of operations, a water-absorbing resin powder (2) in amorphous and crushed form of which particle diameter is 150 μm or more but less than 850 μm was obtained. The solids content in the water-absorbing resin powder (2) was 97% by weight.

Subsequently, by homogeneously mixing 100 parts by weight of the water-absorbing resin powder (2) with 3.52 parts by weight of a surface crosslinking agent solution containing 0.02 parts by weight of ethylene glycol diglycidyl ether, 0.5 parts by weight of propylene glycol, 2.7 parts by weight of ion exchange water, and 0.3 parts by weight of 1,4-butane diol and heating them for 40 minutes at 195°C and classification using a JIS standardized sieve with opening size of 850 μm, a surfaced crosslinked water-absorbing resin particle (2) was obtained. The water-absorbing resin particle (2) has a weight average particle diameter (D50) of 367 μm.

Initial color and color with the lapse of time were measured for the water-absorbing resin particle (2) which was obtained according to the above operations. The results are shown in Table 2.

[Example 5]

Except that water-absorbing resin particle (1) (without chelating agent) of Example 1 was changed to the water-absorbing resin particle (2) (chelating agent was added at 50 ppm during polymerization), the same operations as in Example 1 (granulation using 1.1 parts by weight of aqueous solution of chelating agent, disintegration using a paint shaker, and removal of glass beads) were performed to obtain a particulate water-absorbing agent (5).

The weight average particle diameter (D50) of the water-absorbing resin granulate (5) after the addition of a chelating agent was 376 μm (1.02 times the weight average particle diameter (D50) of the water-absorbing resin particle (2)), and the weight average particle diameter (D50) of the particulate water-absorbing agent (5) was 347 μm (0.92 time the weight average particle diameter (D50) of the water-absorbing resin granulate (5), and 0.95 time the weight average particle diameter (D50) of the water-absorbing resin particle (2)). Meanwhile, physical properties like moisture content are shown in Table 1.

Furthermore, in the above particulate water-absorbing agent (5), the ratio of particles with particle diameter 150 μm or more but less than 850 μm, in which the particle diameter is specified by a JIS standardized sieve, was 97.8° by weight. The amount of deteriorated solubles for different particle sizes and particle distribution are shown in Table 1. Meanwhile, the amount of deteriorated solubles in the entire particulate water-absorbing agent (5) was 21.3% by weight. Furthermore, initial color and color with the lapse of time were measured for particulate water-absorbing agent (5). The results are shown in Table 2.

[Example 6]

Except for having an additional operation in which 1.5 parts by weight of 10% by weight aqueous solution of hydrogen sulfite was added dropwise as an inorganic reducing agent to 100 parts by weight of the water-absorbing resin granulate (5) after addition of a chelating agent followed by homogeneous mixing, the same operations as in Example 5 (disintegration using a paint shaker, and removal of glass beads) were performed to obtain a particulate water-absorbing agent (6).

The weight average particle diameter (D50) of the water-absorbing resin granulate (6) after the addition of a chelating agent and an inorganic reducing agent was 380 μm (1.04 times the weight average particle diameter (D50) of the water-absorbing resin particle (2)), and the weight average particle diameter (D50) of the particulate water-absorbing agent (6) was 345 μm (0.91 time the weight average particle diameter (D50) of the water-absorbing resin granulate (6), and 0.94 time the weight average particle diameter (D50) of the water-absorbing resin particle (2)). Meanwhile, physical properties like moisture content are shown in Table 1.

Furthermore, in the above particulate water-absorbing agent (6), the ratio of particles with particle diameter 150 μm or more but less than 850 μm, in which the particle diameter is specified by a JIS standardized sieve, was 97.8% by weight. The amount of deteriorated solubles for different particle sizes and particle distribution are described in Table 1. Meanwhile, the amount of deteriorated solubles in the entire particulate water-absorbing agent (6) was 21.0% by weight. Furthermore, initial color and color with the lapse of time were measured for particulate water-absorbing agent (6). The results are shown in Table 2.

Meanwhile, the reducing agent content for different particle sizes (B1/B2 = 2.02) was also within a preferred range. Herein, amongst the particles constituting the particulate absorbing agent (6), B1 indicates a reducing agent content (ppm) which is present in particles with a particle diameter of less than 300 μm, in which the particle diameter is specified by a JIS standardized sieve, and B2 indicates a reducing agent content (ppm) which is present in particles with a particle diameter of 300 μm or more but less than 850 μm, in which the particle diameter is specified by a JIS standardized sieve. The reducing agent content was measured by, after extracting it from a particulate water-absorbing agent with physiological saline, HPLC by using the method described in Chem. Pharm. Bull. Vol. 29, P. 3755-3757 (1987).

[Example 7]

By dry mixing of 0.3 part by weight of silicon dioxide (Aerosil 200; Aerosil Japan) as water insoluble inorganic fine particles with 100 parts by weight of the particulate water-absorbing agent (5) obtained from Example 5, a particulate water-absorbing agent (7) was obtained.

The weight average particle diameter (D50) of the particulate water-absorbing agent (7) was 346 μm (0.92 time the weight average particle diameter (D50) of the water-absorbing resin granulate (5), and 0.94 time the weight average particle diameter (D50) of the water-absorbing resin particle (2)). Meanwhile, physical properties like moisture content are shown in Table 1.

Furthermore, in the above particulate water-absorbing agent (7), the ratio of particles with particle diameter 150 μm or more but less than 850 μm, in which the particle diameter is specified by a JIS standardized sieve, was 97.8% by weight. The amount of deteriorated solubles for different particle sizes and particle distribution are shown in Table 1. Meanwhile, the amount of deteriorated solubles in the entire particulate water-absorbing agent (7) was 21.3% by weight. Furthermore, the particulate water-absorbing agent (7) was confirmed to have an improveed blocking ratio after moisture absorption (Example 5; 100% by weight → Example 7; 0° by weight).

Meanwhile, the silicon dioxide content for different particle sizes was 2.01 (= B1/B2) for the particulate water-absorbing agent (7). The silicon dioxide content was measured by, after extracting it from a particulate water-absorbing agent, molybdenum blue spectrophotometric method.

[Example 8]

Except for having, in Example 5, an additional operation in which 1.0 part by weight of 15% by weight aqueous solution of camellia leaf extract (FS-89MO manufactured by SHIRAIMATSU PHARMACEUTICAL Co., LTD) was added dropwise as a water soluble deodorizing agent to 100 parts by weight of the water-absorbing resin granulate (5) after addition of a chelating agent followed by homogeneous mixing, the same operations as in Example 5 (disintegration using a paint shaker, and removal of glass beads) were performed to obtain a particulate water-absorbing agent (8).

The weight average particle diameter (D50) of the water-absorbing resin granulate (8) after the addition of a chelating agent and a water insoluble deodorizing agent was 379 μm (1.03 times the weight average particle diameter (D50) of the water-absorbing resin particle (2)), and the weight average particle diameter (D50) of the particulate water-absorbing agent (8) was 345 μm (0.91 time the weight average particle diameter (D50) of the water-absorbing resin granulate (8), and 0.94 time the weight average particle diameter (D50) of the water-absorbing resin particle (2)). Meanwhile, physical properties like moisture content are shown in Table 1.

Furthermore, in the above particulate water-absorbing agent (8), the ratio of particles with particle diameter 150 μm or more but less than 850 μm, in which the particle diameter is specified by a JIS standardized sieve, was 97.8% by weight. The amount of deteriorated solubles for different particle sizes and particle distribution are shown in Table 1. Meanwhile, the amount of deteriorated solubles in the entire particulate water-absorbing agent (8) was 20.9% by weight.

Furthermore, improved deodorizing property was confirmed for the particulate water-absorbing agent (8). Namely, the result of the malodor test was "×" in Example 5, but it was improved in Example 8 showing "O."

[Example 9]

Except having, in Example 5, an additional operation in which 1.2 parts by weight of aluminum sulfate mixed solution containing 1.0 part by weight of 27% by weight aqueous solution of aluminum sulfate, 0.3 part by weight of 60% by weight aqueous solution of sodium lactate and 0.025 part by weight of polypropylene glycol was added dropwise to 100 parts by weight of the water-absorbing resin granulate (5) after addition of a chelating agent followed by homogeneous mixing, the same operations as in Example 5 (disintegration using a paint shaker, and removal of glass beads) were performed to obtain a particulate water-absorbing agent (9).

The weight average particle diameter (D50) of the water-absorbing resin granulate (9) after the addition of a chelating agent and a polyvalent metal salt was 380 $\mu$m (1.04 times the weight average particle diameter (D50) of the aqueous resin particle (2)), and the weight average particle diameter (D50) of the particulate water-absorbing agent (9) was 345 $\mu$m (0.91 time the weight average particle diameter (D50) of the water-absorbing resin granulate (9), and 0.94 time the weight average particle diameter (D50) of the water-absorbing resin particle (2)). Meanwhile, physical properties like moisture content are shown in Table 1.

Furthermore, in the above particulate water-absorbing agent (9), the ratio of particles with particle diameter 150 $\mu$m or more but less than 850 $\mu$m, in which the particle diameter is specified by a JIS standardized sieve, was 97.8% by weight. The amount of deteriorated solubles for different particle sizes and particle distribution are shown in Table 1. Meanwhile, the amount of deteriorated solubles in the entire particulate water-absorbing agent (9) was 18.9% by weight.

Furthermore, the aluminum content for different particle sizes was 2.03 (= B1/B2) for the particulate water-absorbing agent (9), and an improved SFC (Example 5; 0 $\rightarrow$ Example 9; 10) and an improved blocking ratio after moisture absorption (Example 5; 100% by weight $\rightarrow$ Example 9; 0% by weight) were confirmed. Meanwhile, the aluminum content was measured based on "(G) Extraction rate of polyvalent metal components (% by weight), method for quantifying polyvalent metal components contained in water-absorbing resin" which is disclosed in the U.S. 7,960,269.

[Example 10]

To 100 parts by weight of the water-absorbing resin particle (2) obtained from Preparation Example 2, a mixed solution containing 0.1 part by weight of diethylene triamine pentaacetic acid·trisodium, 0.05 part by weight of propylene glycol, and 1.5 parts by weight of ion exchange water was added dropwise followed by homogenous mixing. Next, the same operations as in Example 1 (granulation using 1.1 parts by weight aqueous solution of chelating agent, disintegration using a paint shaker, and removal of glass beads) were performed to obtain a water-absorbing resin granulate (10) and a particulate water-absorbing agent (10).

The weight average particle diameter (D50) of the water-absorbing resin granulate (10) after the addition of a chelating agent and polyol was 376 $\mu$m (1.02 times the weight average particle diameter (D50) of the aqueous resin particle (2)), and the weight average particle diameter (D50) of the particulate water-absorbing agent (10) was 347 $\mu$m (0.92 time the weight average particle diameter (D50) of the water-absorbing resin granulate (8), and 0.95 time the weight average particle diameter (D50) of the water-absorbing resin agent (2)). Meanwhile, physical properties like moisture content are shown in Table 1.

Furthermore, in the above particulate water-absorbing agent (10), the ratio of particles with particle diameter 150 $\mu$m or more but less than 850 $\mu$m, in which the particle diameter is specified by a JIS standardized sieve, was 97.8% by weight. The amount of deteriorated solubles for different particle sizes and particle distribution are shown in Table 1. Meanwhile, the amount of deteriorated solubles in the entire particulate water-absorbing agent (10) was 19.3% by weight.

[Example 11]

To 100 parts by weight of the water-absorbing resin particle (2) obtained from Preparation Example 2, a mixed solution containing 0.1 part by weight of triethylene tetraamine hexaacetic acid and 1 part by weight of 1 N aqueous solution of sodium hydroxide was added dropwise followed by homogenous mixing. Next, the same operations as in Example 1 (granulation using 1.1 parts by weight aqueous solution of chelating agent, disintegration using a paint shaker, and removal of glass beads) were performed to obtain a water-absorbing resin granulate (11) and a particulate water-absorbing agent (11).

The weight average particle diameter (D50) of the water-absorbing resin granulate (11) after the addition of a chelating agent was 380 $\mu$m (1.04 times the weight average particle diameter (D50) of the aqueous resin particle (2)), and the weight average particle diameter (D50) of the particulate water-absorbing agent (11) was 345 $\mu$m (0.91 time the weight average particle diameter (D50) of the water-absorbing resin granulate (11), and 0.94 time the weight average particle diameter (D50) of the water-absorbing resin particle (2)). Meanwhile, physical properties like moisture content are shown in Table 1.

Furthermore, in the above particulate water-absorbing agent (11), the ratio of particles with particle diameter

150 μm or more but less than 850 μm, in which the particle diameter is specified by a JIS standardized sieve, was 97.8% by weight. The amount of deteriorated solubles for different particle sizes and particle distribution are shown in Table 1. Meanwhile, the amount of deteriorated solubles in the entire particulate water-absorbing agent (11) was 17.8% by weight.

[Preparation Example 3]

Except that the amount of ethylene glycol diglycidyl ether that was used was changed to 0.024 parts by weight relative to 100 parts by weight of the water-absorbing resin powder (2) and also the heating treatment was performed for 40 minutes at 176°C, the same operations as in Preparation Example 2 were performed to obtain a particulate water-absorbing agent (3). The weight average particle diameter (D50) of the water-absorbing resin particle (3) was 355 μm.

[Example 12]

Except that the water-absorbing resin particle (1) of Example 1 was changed to the water-absorbing resin particle (3), the same operations as in Example 1 (granulation using 1.1 parts by weight aqueous solution of chelating agent, disintegration using a paint shaker, and removal of glass beads) were performed to obtain a water-absorbing resin granulate (12). By dry mixing of 0.3 part by weight of silicon dioxide (Aerosil 200; Aerosil Japan) as water insoluble inorganic fine particles with 100 parts by weight of the obtained particulate water absorbent granulate (12), a particulate water-absorbing agent (12) was obtained.

The weight average particle diameter (D50) of the water-absorbing resin granulate (12) after the addition of a chelating agent was 370 μm (1.04 times the weight average particle diameter (D50) of the aqueous resin particle (3)), and the weight average particle diameter (D50) of the particulate water-absorbing agent (12) was 340 μm (0.92 time the weight average particle diameter (D50) of the water-absorbing resin granulate (12), and 0.96 time the weight average particle diameter (D50) of the water-absorbing resin particle (3)). Meanwhile, physical properties like moisture content are shown in Table 1.

Furthermore, in the above particulate water-absorbing agent (12), the ratio of particles with particle diameter 150 μm or more but less than 850 μm, in which the particle diameter is specified by a JIS standardized sieve, was 98.5% by weight. The amount of deteriorated solubles for different particle sizes and particle distribution are shown in Table 1. Meanwhile, the amount of deteriorated solubles in the entire particulate water-absorbing agent (12) was 22.1% by weight. Furthermore, the particulate water-absorbing agent (12) was confirmed to have an improved blocking ratio after moisture absorption (Example 1; 100% by weight → Example 12; 0° by weight).

[Preparation Example 4]

In Preparation Example 2, except for homogeneously mixing 100 parts by weight of the water-absorbing resin powder (2) with a surface crosslinking agent solution containing 0.024 part by weight of ethylene glycol diglycidyl ether, 0.52 part by weight of propylene glycol, 2.1 parts by weight of ion exchange water, and 0.31 part by weight of ethylene carbonate and heating the mixture for 40 minutes at 176°C, a water-absorbing resin particle (4) was obtained according to the same operations as in Preparation Example 2. The water-absorbing resin particle (4) has a weight average particle diameter (D50) of 356 μm.

[Example 13]

Except that the water-absorbing resin particle (1) of Example 1 was changed to the water-absorbing resin particle (4), the same operations as in Example 1 (granulation using 1.1 parts by weight aqueous solution of chelating agent, disintegration using a paint shaker, and removal of glass beads) were performed to obtain a water-absorbing resin granulate (13). By dry mixing of 0.3 part by weight of silicon dioxide (Aerosil 200; Aerosil Japan) as water insoluble inorganic fine particles with 100 parts by weight of the obtained particulate water absorbent granulate (13), a particulate water-absorbing agent (13) was obtained.

The weight average particle diameter (D50) of the water-absorbing resin granulate (13) after the addition of a chelating agent was 374 μm (1.05 times the weight average particle diameter (D50) of the aqueous resin particle (4)), and the weight average particle diameter (D50) of the particulate water-absorbing agent (13) was 340 μm (0.91 time the weight average particle diameter (D50) of the water-absorbing resin granulate (13), and 0.96 time the weight average particle diameter (D50) of the water-absorbing resin particle (4)). Meanwhile, physical properties like moisture content are shown in Table 1.

Furthermore, in the above particulate water-absorbing agent, the ratio of particles with particle diameter 150 μm or more but less than 850 μm, in which the particle diameter is specified by a JIS standardized sieve, was 98.5% by weight. The amount of deteriorated solubles for different particle sizes and particle distribution are shown in Table 1.

Meanwhile, the amount of deteriorated solubles in the entire particulate water-absorbing agent (13) was 23.9% by weight. Furthermore, the particulate water-absorbing agent (13) was confirmed to have an improved blocking ratio after moisture absorption (Example 1; 100% by weight → Example 13; 0° by weight).

[Example 14]

Except that the chelating agent is changed to 0.1 parts by weight of ethylene diamine tetra(methylenephosphonic acid) (EDTMP) -pentasodium, the same operations as in Example 1 (disintegration using a paint shaker, and removal of glass beads) were performed to obtain a particulate water-absorbing agent (14).

Physical properties of the obtained particulate water-absorbing agent (14) were as follows. CRC; 45 (g/g), AAP; 27 (g/g), moisture content; 5.5% by weight, C1/C2; 2.73, an increased amount of deteriorated solubles; particles that are less than 300 $\mu$m: 11.1% by weight, particles that are 300 $\mu$m or more but less than 850 $\mu$m: 14.2% by weight.

[Comparative Example 3]

The water-absorbing resin particle (2) obtained from Preparation Example 2 (chelating agent is added at 0.005% by mass during polymerization (relative to monomer solids content)) was used as the comparative particulate water-absorbing agent (3).

Physical properties of the obtained comparative particulate water-absorbing agent (3) are as follows. CRC; 39 (g/g), AAP; 29 (g/g), moisture content; 2.9% by weight, C1/C2; 1.05, an increased amount of deteriorated solubles; particles that are less than 300 $\mu$m: 17.2% by weight, particles that are 300 $\mu$m or more but less than 850 $\mu$m: 15.0% by weight.

[Table 1]

| | | Example 1 | Example 2 | Example 3 | Comparative Example 1 | Comparative Example 2 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|---|---|
| Water-absorbing resin particles D50 | [μm] | (1) 446 | (1) 446 | (1) 446 | (1) 446 | (1) 446 | (1) 446 | (2) 367 | (2) 367 | (2) 367 | (2) 367 | (2) 367 | (2) 367 | (2) 367 | (3) 355 | (4) 356 |
| Chelating agent [1] | [Parts by weight] | DTPA 0.1 | DTPA 0.05 | DTPA 0.003 | DTPA 0.003 | DTPA 0.002 | DTPA 0.01/0.025 | DTPA 0.1 | DTPA 0.1 | DTPA 0.1 | DTPA 0.1 | DTPA 0.1 | DTPA 0.1 | TTHA 0.1 | DTPA 0.1 | DTPA 0.1 |
| Water-absorbing resin granulate D50 | [μm] | (1) 477 | (2) 480 | (3) 479 | Comparative Example (1) 479 | Comparative Example (2) 479 | - - | (4) 376 | (5) 380 | (4) 376 | (6) 379 | (7) 380 | (8) 376 | (9) 380 | (10) 370 | (11) 374 |
| Paint shaker | | ○ | ○ | ○ | × | × | × | ○ | ○ | ○ | ○ | ○ | ○ | ○ | ○ | ○ |
| Particulate water-absorbing agent | | (1) | (2) | (3) | Comparative Example (1) | Comparative Example (2) | (4) | (5) | (6) | (7) | (8) | (9) | (10) | (11) | (12) | (13) |
| D50 | [μm] | 430 | 431 | 426 | 479 | 479 | - | 347 | 345 | 346 | 345 | 345 | 347 | 345 | 340 | 340 |
| CRC | [g/g] | 45 | 45 | 45 | 45 | 45 | 45 | 36 | 36 | 36 | 36 | 36 | 36 | 36 | 39 | 41 |
| AAP | [g/g] | 27 | 27 | 27 | 27 | 27 | 27 | 33 | 33 | 30 | 33 | 33 | 33 | 33 | 29 | 27 |
| Moisture content | [wt%] | 5.6 | 5.3 | 5.4 | 5.5 | 5.5 | 5.4 | 4.1 | 5.4 | 4.1 | 5.1 | 5.0 | 4.4 | 4.1 | 4.0 | 4.1 |
| SFC | [*] | - | - | - | - | - | - | 0 | - | - | - | 10 | - | - | - | - |
| Blocking ratio after moisture absorption | [wt%] | 100 | - | - | - | - | - | 100 | - | 0 | - | 0 | - | - | 0 | 0 |

(continued)

| | | Example 1 | Example 2 | Example 3 | Comparative Example 1 | Comparative Example 2 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|---|---|
| Content for different particle sizes | | | | | | | | | | | | | | | | |
| Particle diameter 300 ↓ [2] | [wt%] | 17 | 17 | 17 | 12 | 12 | 16 | 32 | 33 | 33 | 32 | 33 | 30 | 35 | 38 | 37 |
| Particle diameter 300-850 [2] | [wt%] | 83 | 83 | 83 | 88 | 88 | 84 | 68 | 67 | 67 | 68 | 67 | 70 | 65 | 62 | 63 |
| Chelating agent content | | | | | | | | | | | | | | | | |
| Particle diameter 300 ↓ [2] (C1) | [ppm] | 2471 | 1082 | 61 | 30 | 21 | 239 | 1650 | 1668 | 1688 | 1588 | 1544 | 1820 | 1714 | 1256 | 1454 |
| Particle diameter 300-850 [2] (C2) | [ppm] | 891 | 399 | 27 | 31 | 21 | 93 | 830 | 832 | 840 | 721 | 765 | 733 | 706 | 753 | 803 |
| C1/C2 | | 2.77 | 2.71 | 2.26 | 0.97 | 1.00 | 2.57 | 1.99 | 2.00 | 2.01 | 2.20 | 2.02 | 2.48 | 2.43 | 1.67 | 1.81 |
| Amount of soluble contents | | | | | | | | | | | | | | | | |
| Particle diameter 300 ↓ [2] | [wt%] | 15.7 | 16.3 | 17.7 | 18.0 | 18.2 | 18.0 | 10.2 | 9.9 | 10.4 | 10.5 | 10.8 | 10.1 | 8. 9 | 12.5 | 14.2 |
| Particle diameter 300-850 [2] | [wt%] | 13.5 | 14.0 | 14.5 | 14.3 | 15.0 | 14.0 | 9.0 | 8. 7 | 9.2 | 8. 9 | 8.7 | 9.3 | 8.2 | 10.2 | 12.1 |

37

(continued)

| | | Exam-ple 1 | Exam-ple 2 | Exam-ple 3 | Compara-tive Exam-ple 1 | Compara-tive Exam-ple 2 | Example 4 | Exam-ple 5 | Exam-ple 6 | Exam-ple 7 | Exam-ple 8 | Exam-ple 9 | Exam-ple 10 | Exam-ple 11 | Exam-ple 12 | Exam-ple 13 |
|---|---|---|---|---|---|---|---|---|---|---|---|---|---|---|---|---|
| Amount of deteriorat-ed solubles | | | | | | | | | | | | | | | | |
| Total | [wt%] | 27.2 | 27.5 | 30.6 | 30.9 | 51.4 | 28.8 | 21.3 | 21.0 | 21.3 | 20.9 | 18.9 | 19.3 | 17.8 | 22.1 | 23.9 |
| Particle di-ameter 300 ↓ 2) | [wt%] | 26.8 | 27.0 | 29.5 | 36.0 | 76.0 | 27.9 | 19.7 | 19.5 | 19.8 | 19.3 | 18.4 | 18.2 | 15.1 | 21.7 | 23.2 |
| Particle di-ameter 300-850 2) | [wt%] | 27.3 | 27.6 | 30.8 | 30.2 | 48.0 | 29.0 | 22.0 | 21.8 | 22.1 | 21.6 | 19.1 | 19.7 | 19.2 | 22.4 | 24.3 |
| Increased amount of deteriorat-ed solubles | | | | | | | | | | | | | | | | |
| Particle di-ameter 300 ↓ 2) | [wt%] | 11.1 | 10.7 | 11.8 | 18.0 | 57.8 | 9. 9 | 9.5 | 9.6 | 9.4 | 8.8 | 7.6 | 8.1 | 6.2 | 9.2 | 9.0 |
| Particle di-ameter 300-850 2) | [wt%] | 13.8 | 13.6 | 16.3 | 15.9 | 33.0 | 15.0 | 13.0 | 13.1 | 12.9 | 12.7 | 10.4 | 10.4 | 11.0 | 12.2 | 12.2 |
| 1) DTPA; Diethylene triamine pentaacetic acid · trisodium<br>TTHA; Triethylene tetraamine hexaacetic acid<br>2) Respectively representing: Particle diameter 300↓: Particles with the particle diameter of less than 300 $\mu$m; Particle diameter 300-850: Particles with the particle diameter of 300 $\mu$m or more but less than 850 $\mu$m.<br>(*) Unit of SFC; [$\times$ 10$^{-7}$·cm$^3$·s·g$^{-1}$] | | | | | | | | | | | | | | | | |

EP 3 056 268 B1

(Summary)

As shown in the above Table 1, by adjusting the chelating agent content depending on different particle sizes, a variation in gel stability can be suppressed and also high water absorption performance can be obtained.

Namely, as shown in Comparative Example 1, when the chelating agent amount (C1) contained in particles with the particle diameter of less than 300 μm and the chelating agent amount (C2) contained in particles with the particle diameter of 300 μm or more but less than 850 μm are similar to each other (C1/C2 is about 1), the increased amount of deteriorated solubles in the particles with the particle diameter of 300 μm or more but less than 850 μm can be suppressed but the increased amount of deteriorated solubles in the particles with the particle diameter of less than 300 μm cannot be sufficiently suppressed.

Furthermore, in Comparative Example 1, a difference between the amount of deteriorated solubles in the particles with the particle diameter of less than 300 μm and the amount of deteriorated solubles in the particles with the particle diameter of 300 μm or more but less than 850 μm was high (5.8% by weight = 36.0 - 30.2). Thus, for example, due to a variation in particle size of the particulate water-absorbing agent, the amount of deteriorated solubles is varied greatly.

Meanwhile, it was found that, when the ratio of the above C1 and C2 (C1/C2) is controlled to satisfy Formula (1) (Examples 1 to 5), there was no influence of the particle diameter and thus excellent gel stability is exhibited. [Mathematical 16]

$$1.2 < C1/C2 < 4.0 \ … \ (1)$$

Namely, in Example 3, a difference between the amount of deteriorated solubles in the particles with the particle diameter of less than 300 μm and the amount of deteriorated solubles in the particles with the particle diameter of 300 μm or more but less than 850 μm was small (1.3% by weight = 30.8 - 29.5) even when the chelating agent amount was the same as that in Comparative Example 1 (chelating agent: 0.003 part by weight), and thus a variation in the amount of deteriorated solubles in the particulate water-absorbing agent was small. It was also found that the amount of deteriorated solubles in the entire water-absorbing agent was improved in Example 3 as compared to Comparative Example 1 (Example 3; 30.6% by weight, Comparative Example 1; 30.9% by weight).

When Examples 1 to 3 and 5 to 13 are compared to Comparative Examples 1 and 2, it was found that, according to disintegration of the water-absorbing resin granulate, which has been in granulate form due to aggregation of particles, with a paint shaker, a water-absorbing agent having good water absorption performance including high gel stability and little variation in gel stability amongst different particle sizes can be obtained more efficiently.

Furthermore, when Comparative Example 1 (0.003 parts by weight of chelating agent) is compared to Comparative Example 2 (0.002 parts by weight of chelating agent), and Example 1 (0.1 parts by weight of chelating agent), Example 2 (0.05 parts by weight of chelating agent), and Example 3 (0.003 parts by weight of chelating agent) are compared to one another, it was found that, as the chelating agent amount is increased to a predetermined amount or more, not only the amount of deteriorated solubles but also a difference between the amount of deteriorated solubles in the particles with the particle diameter of less than 300 μm and the amount of deteriorated solubles in the particles with the particle diameter of 300 μm or more but less than 850 μm decreases.

[Table 2]

| | Chelating agent content [wt%] | Reducing agent content [wt%] | Initial color | | | Color with the lapse of time | | |
|---|---|---|---|---|---|---|---|---|
| | | | L | a | b | L | a | b |
| Water-absorbing resin particle (2) | 0 | 0 | 90.09 | -0.02 | 6.76 | 68.80 | 4.09 | 14.52 |
| Particulate water-absorbing agent (5) | 0.1 | 0 | 90.04 | -0.06 | 6.74 | 73.68 | 3.20 | 12.75 |
| Particulate water-absorbing agent (6) | 0.1 | 0.15 | 90.39 | -0.42 | 6.52 | 82.89 | 0.99 | 8.61 |

(Summary)

With the particulate water-absorbing agent (6), coloration was improved compared to Preparation Example 2 (without any additives) or the particulate water-absorbing agent (5) (only with chelating agent).

Industrial Applicability

The particulate water-absorbing agent of the present invention is preferably applied to a use in which a dehydrating property and a water-retaining property are required, that is, a water-absorbing agent of a hygienic material like disposable diaper and sanitary napkin, a water-retaining agent for medical use, a water-retaining agent for agricultural and horticultural use, and other various industrial dehydrating agents or the like.

**Claims**

1. A particulate water-absorbing agent comprising a water-absorbing resin as a main component and a chelating agent, the water-absorbing agent containing particles with a particle diameter of less than 300 μm and particles with a particle diameter of 300 μm or more but less than 850 μm, as specified by a Japanese Industrial Standard (JIS) standardized sieve,

   wherein an amount of the chelating agent included is 0.001 to 5 parts by weight per 100 parts by weight of a water-absorbing resin in solid and satisfies Formula (1):
   [Mathematical 1]

$$1.2 < C1/C2 < 4.0 \ldots (1)$$

   where, in Formula (1), C1 indicates that an amount (ppm) of the chelating agent present in the particles of a particle diameter of less than 300 μm, as specified by a JIS standardized sieve, amongst particle components for the particulate water-absorbing agent, and C2 indicates that an amount (ppm) of the chelating agent present in the particles of a particle diameter of 300 μm or more but less than 850 μm, as specified by a JIS standardized sieve, amongst particle components for the particulate water-absorbing agent.

2. The particulate water-absorbing agent according to claim 1, wherein the amount of the chelating agent included is 0.03 to 0.9 parts by weight per 100 parts by weight of the water-absorbing resin in solid.

3. The particulate water-absorbing agent according to claim 1 or 2, wherein the chelating agent is at least one member selected from the group consisting of: amino polyvalent carboxylic acid, organic polyvalent phosphoric acid, inorganic polyvalent phosphoric acid, amino polyvalent phosphoric acid and a tropolone derivative.

4. The particulate water-absorbing agent according to any one of claims 1 to 3, wherein:

   1) an increased amount for deteriorated soluble particles having a particle diameter of less than 300 μm, as specified by a JIS standardized sieve, is 0 to 15% by weight, and
   2) an increased amount for deteriorated soluble particles having a particle diameter of 300 μm or more but less than 850 μm, as specified by a JIS standardized sieve, is 0 to 20% by weight,
   wherein an increased amount for deteriorated soluble particles is a value defined by Formula (2):
   [Mathematical 2]

```
Increased amount for deteriorated soluble particles (% by
weight) = (Amount of soluble particles for 1 hour in
deterioration test solution) - (Amount of soluble particles
for 1 hour in physiological saline) … (2)
```

   where, in Formula 2, the deterioration test solution means physiological saline to which 0.1% by weight of L-ascorbic acid is added.

5. The particulate water-absorbing agent according to any one of claims 1 to 4, wherein

   a ratio of particles with a particle diameter of 150 μm or more but less than 850 μm, as specified by a Japanese Industrial Standard (JIS) standardized sieve, is 90% by weight or more, and
a weight ratio between particles with a particles diameter of less than 300 µm and particles with a particle diameter of 300 µm or more but less than 850 µm, as specified by a Japanese Industrial Standard (JIS) standardized sieve, is 70 : 30 to 10 : 90.

6. The particulate water-absorbing agent according to any one of claims 1 to 5 further comprising an inorganic reducing agent.

7. The particulate water-absorbing agent according to claim 6, wherein the inorganic reducing agent contains a reductive sulfur atom.

8. The particulate water-absorbing agent according to any one of claims 1 to 7 further comprising water-insoluble inorganic fine particles.

9. The particulate water-absorbing agent according to any one of claims 1 to 8 further comprising a water-soluble deodorizing agent.

10. The particulate water-absorbing agent according to any one of claims 1 to 9 further comprising a polyvalent metal salt and/or a cationic polymer.

11. The particulate water-absorbing agent according to any one of claims 1 to 10 further comprising polyol.

12. The particulate water-absorbing agent according to any one of claims 1 to 11 further comprising an iron ion, wherein an amount of the iron ion is 0.001 to 2 ppm.

13. The particulate water-absorbing agent according to any one of claims 1 to 12, wherein the particulate water-absorbing agent has: a fluid retention capacity without pressure (CRC) including moisture content correction to be 30 (g/g) or more against physiological saline, and fluid retention capacity under pressure at a pressure of 2.06 kPa (AAP) including moisture content correction to be 20 (g/g) or more against physiological saline.

14. The particulate water-absorbing agent according to any one of claims 1 to 13, wherein a moisture content obtained from an amount lost from drying at 180°C for 3 hours is 0.5 to 15% by weight.

15. A method for producing a particulate water-absorbing agent comprising a water-absorbing resin as a main component and a chelating agent, the water-absorbing agent containing particles with a particle diameter of less than 300 µm and particles with a particle diameter of 300 µm or more but less than 850 µm, as specified by a Japanese Industrial Standard (JIS) standardized sieve,

wherein an amount of the chelating agent included is 0.001 to 5 parts by weight per 100 parts by weight of a water-absorbing resin in solid and satisfies Formula (1):
[Mathematical 3]

$$1.2 < C1/C2 < 4.0 \ldots (1)$$

where, in Formula (1), C1 indicates that an amount (ppm) of the chelating agent present in the particles of a particle diameter of less than 300 µm, as specified by a JIS standardized sieve, amongst particle components for the particulate water-absorbing agent, and C2 indicates that an amount (ppm) of the chelating agent present in the particles of a particle diameter of 300 µm or more but less than 850 µm, as specified by a JIS standardized sieve, amongst particle components for the particulate water-absorbing agent; and
wherein the method comprises one of the following a) and b):

a) the steps of:

adding a chelating agent to a surface-crosslinked water-absorbing resin to obtain a water-absorbing resin granule, of which weight average particle diameter (D50) is increased to 1.01 to 10 times relative to the weight average particle diameter before adding the chelating agent; and
crushing the water-absorbing resin granule and reducing the weight average particle diameter (D50) by 5% or more to obtain a particulate water-absorbing agent; and

b) the steps of:

sizing a surface-crosslinked water-absorbing resin into a plurality of different particle sizes;
adding a chelating agent as per the plurality of the different particle sizes; and
re-mixing a plurality of the water-absorbing resins, to which said chelating agent was added, to obtain a particulate water-absorbing agent.

**16.** The method according to claim 15, wherein the chelating agent in a state of being dissolved and/or dispersed into a mixed solvent containing water and a hydrophilic organic solvent is added to the water-absorbing resin particles to obtain a particulate water-absorbing agent.

**17.** The method according to claim 16, wherein the hydrophilic organic solvent is polyol.

**18.** An absorbent article comprising a particulate water-absorbing agent as set forth in any one of claims 1 to 14.

**Patentansprüche**

**1.** Teilchenförmiges wasserabsorbierendes Mittel, umfassend ein wasserabsorbierendes Harz als eine Hauptkomponente und einen Chelatbildner, wobei das wasserabsorbierende Mittel Teilchen mit einem Teilchendurchmesser von weniger als 300 $\mu$m und Teilchen mit einem Teilchendurchmesser von 300 $\mu$m oder mehr, aber weniger als 850 $\mu$m, wie mit einem genormten Sieb gemäß Japanese Industrial Standard (JIS) bestimmt, enthält,

wobei eine enthaltene Menge des Chelatbildners 0,001 bis 5 Gewichtsteile pro 100 Gewichtsteile eines wasserabsorbierenden Harzes in fester Form beträgt und Formel (1) erfüllt:
[Mathematische Formel 1]

$$1{,}2 < C1/C2 < 4{,}0 \ldots (1)$$

wobei in Formel (1) C1 anzeigt, dass in den Teilchen mit einem Teilchendurchmesser von weniger als 300 $\mu$m, wie mit einem genormten Sieb gemäß JIS bestimmt, unter Teilchenkomponenten für das teilchenförmige wasserabsorbierende Mittel eine Menge (ppm) des Chelatbildners vorliegt, und C2 anzeigt, dass in den Teilchen mit einem Teilchendurchmesser von 300 $\mu$m oder mehr, aber weniger als 850 $\mu$m, wie mit einem genormten Sieb gemäß JIS bestimmt, unter Teilchenkomponenten für das teilchenförmige wasserabsorbierende Mittel eine Menge (ppm) des Chelatbildners vorliegt.

**2.** Teilchenförmiges wasserabsorbierendes Mittel nach Anspruch 1, wobei die enthaltene Menge des Chelatbildners 0,03 bis 0,9 Gewichtsteile pro 100 Gewichtsteile des wasserabsorbierenden Harzes in fester Form beträgt.

**3.** Teilchenförmiges wasserabsorbierendes Mittel nach Anspruch 1 oder 2, wobei es sich bei dem Chelatbildner um mindestens ein Mitglied aus der Gruppe bestehend aus einer mehrwertigen Aminocarbonsäure, einer mehrwertigen organischen Phosphorsäure, einer mehrwertigen anorganischen Phosphorsäure, einer mehrwertigen Aminophosphorsäure und einem Tropolonderivat handelt.

**4.** Teilchenförmiges wasserabsorbierendes Mittel nach einem der Ansprüche 1 bis 3, wobei:

1) eine erhöhte Menge für verschlechterte lösliche Teilchen mit einem Teilchendurchmesser von weniger als 300 $\mu$m, wie mit einem genormten Sieb gemäß JIS bestimmt, 0 bis 15 Gew.-% beträgt und
2) eine erhöhte Menge für verschlechterte lösliche Teilchen mit einem Teilchendurchmesser von 300 $\mu$m oder mehr, aber weniger als 850 $\mu$m, wie mit einem genormten Sieb gemäß JIS bestimmt, 0 bis 20 Gew.-% beträgt, wobei eine erhöhte Menge für verschlechterte lösliche Teilchen ein durch Formel (2) definierter Wert ist:
[Mathematische Formel 2]

```
Erhöhte Menge für verschlechterte lösliche Teilchen
(Gew.-%) = (Menge an löslichen Teilchen für 1 Stunde
in   Verschlechterungstestlösung)   -   (Menge   an
löslichen Teilchen für 1 Stunde in physiologischer
Kochsalzlösung) … (2)
```

wobei in Formel 2 die Verschlechterungstestlösung physiologische Kochsalzlösung mit Zusatz von 0,1 Gew.-% L-Ascorbinsäure bedeutet.

5. Teilchenförmiges wasserabsorbierendes Mittel nach einem der Ansprüche 1 bis 4, wobei

ein Verhältnis von Teilchen mit einem Teilchendurchmesser von 150 $\mu$m oder mehr, aber weniger als 850 um, wie mit einem genormten Sieb gemäß JIS bestimmt, 90 Gew.-% oder mehr beträgt und
ein Gewichtsverhältnis zwischen Teilchen mit einem Teilchendurchmesser von weniger als 300 $\mu$m und Teilchen mit einem Teilchendurchmesser von 300 $\mu$m oder mehr, aber weniger als 850 $\mu$m, wie mit einem genormten Sieb gemäß JIS bestimmt, 70:30 bis 10:90 beträgt.

6. Teilchenförmiges wasserabsorbierendes Mittel nach einem der Ansprüche 1 bis 5, ferner umfassend ein anorganisches Reduktionsmittel.

7. Teilchenförmiges wasserabsorbierendes Mittel nach Anspruch 6, wobei das anorganische Reduktionsmittel ein reduzierend wirkendes Schwefelatom enthält.

8. Teilchenförmiges wasserabsorbierendes Mittel nach einem der Ansprüche 1 bis 7, ferner umfassend wasserlösliche anorganische feine Teilchen.

9. Teilchenförmiges wasserabsorbierendes Mittel nach einem der Ansprüche 1 bis 8, ferner umfassend ein wasserlösliches Desodorierungsmittel.

10. Teilchenförmiges wasserabsorbierendes Mittel nach einem der Ansprüche 1 bis 9, ferner umfassend ein mehrwertiges Metallsalz und/oder ein kationisches Polymer.

11. Teilchenförmiges wasserabsorbierendes Mittel nach einem der Ansprüche 1 bis 10, ferner umfassend Polyol.

12. Teilchenförmiges wasserabsorbierendes Mittel nach einem der Ansprüche 1 bis 11, ferner umfassend ein Eisenion, wobei eine Menge des Eisenions 0,001 bis 2 ppm beträgt.

13. Teilchenförmiges wasserabsorbierendes Mittel nach einem der Ansprüche 1 bis 12, wobei das teilchenförmige wasserabsorbierende Mittel eine Flüssigkeitsretentionskapazität ohne Druck (CRC) einschließlich Feuchtigkeitsgehaltkorrektur von 30 (g/g) oder mehr gegen physiologische Kochsalzlösung und eine Flüssigkeitsretentionskapazität unter Druck bei einem Druck von 2,06 kPa (AAP) einschließlich Feuchtigkeitsgehaltkorrektur von 20 (g/g) oder mehr gegen physiologische Kochsalzlösung aufweist.

14. Teilchenförmiges wasserabsorbierendes Mittel nach einem der Ansprüche 1 bis 13, wobei ein aus einer durch Trocknung bei 180°C über einen Zeitraum von 3 Stunden verlorenen Menge erhaltener Feuchtigkeitsgehalt 0,5 bis 15 Gew.-% beträgt.

15. Verfahren zur Herstellung eines teilchenförmigen wasserabsorbierenden Mittels, umfassend ein wasserabsorbierendes Harz als eine Hauptkomponente und einen Chelatbildner, wobei das wasserabsorbierende Mittel Teilchen mit einem Teilchendurchmesser von weniger als 300 $\mu$m und Teilchen mit einem Teilchendurchmesser von 300 $\mu$m oder mehr, aber weniger als 850 $\mu$m, wie mit einem genormten Sieb gemäß Japanese Industrial Standard (JIS) bestimmt, enthält,

wobei eine enthaltene Menge des Chelatbildners 0,001 bis 5 Gewichtsteile pro 100 Gewichtsteile eines wasserabsorbierenden Harzes in fester Form beträgt und Formel (1) erfüllt:

[Mathematische Formel 3]

$$1{,}2 < C1/C2 < 4{,}0 \ldots (1)$$

wobei in Formel (1) C1 anzeigt, dass in den Teilchen mit einem Teilchendurchmesser von weniger als 300 μm, wie mit einem genormten Sieb gemäß JIS bestimmt, unter Teilchenkomponenten für das teilchenförmige wasserabsorbierende Mittel eine Menge (ppm) des Chelatbildners vorliegt, und C2 anzeigt, dass in den Teilchen mit einem Teilchendurchmesser von 300 μm oder mehr, aber weniger als 850 μm, wie mit einem genormten Sieb gemäß JIS bestimmt, unter Teilchenkomponenten für das teilchenförmige wasserabsorbierende Mittel eine Menge (ppm) des Chelatbildners vorliegt; und
wobei das Verfahren einen der folgenden Punkte a) und b) umfasst:

    a) die Schritte:

        Zugeben eines Chelatbildners zu einem oberflächenvernetzten wasserabsorbierenden Harz unter Erhalt eines Granulats von wasserabsorbierendem Harz, dessen gewichtsmittlerer Teilchendurchmesser (D50) gegenüber dem gewichtsmittleren Teilchendurchmesser vor der Zugabe des Chelatbildners um das 1,01- bis 10-fache erhöht ist; und
        Zerkleinern des Granulats von wasserabsorbierendem Harz und Verringern des gewichtsmittleren Teilchendurchmessers (D50) um 5 % oder mehr unter Erhalt eines teilchenförmigen wasserabsorbierenden Mittels; und

    b) die Schritte:

        Klassieren eines oberflächenvernetzten wasserabsorbierenden Harzes in mehrere verschiedene Teilchengrößen;
        Zugeben eines Chelatbildners gemäß den mehreren verschiedenen Teilchengrößen und
        Wiedermischen von mehreren der wasserabsorbierenden Harze, denen der Chelatbildner zugesetzt wurde,
        unter Erhalt eines teilchenförmigen wasserabsorbierenden Mittels.

16. Verfahren nach Anspruch 15, wobei der Chelatbildner in Form einer Lösung und/oder Dispersion in einem Mischlösungsmittel, das Wasser und ein hydrophiles organisches Lösungsmittel enthält, zu den Teilchen aus wasserabsorbierendem Harz gegeben wird, um ein teilchenförmiges wasserabsorbierendes Mittel zu erhalten.

17. Verfahren nach Anspruch 16, wobei es sich bei dem hydrophilen organischen Lösungsmittel um Polyol handelt.

18. Absorbierender Artikel, umfassend ein teilchenförmiges wasserabsorbierendes Mittel gemäß einem der Ansprüche 1 bis 14.


**Revendications**

1. Agent particulaire absorbant l'eau comprenant une résine absorbant l'eau en tant que composant principal et un agent chélatant, l'agent absorbant l'eau contenant des particules dotées d'un diamètre de particule de moins de 300 μm et des particules dotées d'un diamètre de particule de 300 μm ou plus mais inférieur à 850 μm, comme spécifié par un tamis normalisé de la norme industrielle japonaise (JIS),

    une quantité de l'agent chélatant inclus étant de 0,001 à 5 parties en poids par 100 parties en poids d'une résine absorbant l'eau en solide et satisfaisant la formule (1) :
    [formule mathématique 1]

$$1{,}2 < C1/C2 < 4{,}0 \ldots (1)$$

où, dans la formule (1), C1 indique une quantité (ppm) de l'agent chélatant présent dans les particules d'un diamètre de particule de moins de 300 μm, comme spécifié par un tamis normalisé de la JIS, parmi des composants de particules pour l'agent particulaire absorbant l'eau, et C2 indique une quantité (ppm) de l'agent chélatant présent dans les particules d'un diamètre de particule de 300 μm ou plus mais inférieur à 850 μm, comme spécifié par un tamis normalisé de la JIS, parmi des composants de particules pour l'agent particulaire absorbant l'eau.

2. Agent particulaire absorbant l'eau selon la revendication 1, la quantité de l'agent chélatant inclus étant de 0,03 à 0,9 partie en poids par 100 parties en poids de la résine absorbant l'eau en solide.

3. Agent particulaire absorbant l'eau selon la revendication 1 ou 2, l'agent chélatant étant au moins un élément choisi dans le groupe constitué par : un acide carboxylique polyvalent amino, un acide phosphorique polyvalent organique, un acide phosphorique polyvalent inorganique, un acide phosphorique polyvalent amino et un dérivé de tropolone.

4. Agent particulaire absorbant l'eau selon l'une quelconque des revendications 1 à 3,

   1) une quantité augmentée pour des particules solubles détériorées possédant un diamètre de particule de moins de 300 μm, comme spécifié par un tamis normalisé de la JIS, étant de 0 à 15 % en poids, et
   2) une quantité augmentée pour des particules solubles détériorées possédant un diamètre de particule de 300 μm ou plus mais inférieur à 850 μm, comme spécifié par un tamis normalisé de la JIS, étant de 0 à 20 % en poids, une quantité augmentée pour des particules solubles détériorées étant une valeur définie par la formule (2) :
   [formule mathématique 2]

   ```
   quantité augmentée pour des particules solubles
   détériorées (% en poids) = (quantité de particules
   solubles pendant 1 heure dans une solution de test
   de détérioration) - (quantité de particules solubles
   pendant 1 heure dans une solution saline
   physiologique) … (2)
   ```

   où, dans la formule 2, la solution de test de détérioration signifie une solution saline physiologique à laquelle 0,1 % en poids de L-acide ascorbique est ajouté.

5. Agent particulaire absorbant l'eau selon l'une quelconque des revendications 1 à 4,

   un rapport de particules dotées d'un diamètre de particule de 150 μm ou plus mais inférieur à 850 μm, comme spécifié par un tamis normalisé de la norme industrielle japonaise (JIS), étant de 90 % en poids ou plus, et un rapport en poids entre des particules dotées d'un diamètre de particule de moins de 300 μm et des particules dotées d'un diamètre de particule de 300 μm ou plus mais inférieur à 850 μm, comme spécifié par un tamis normalisé de la norme industrielle japonaise (JIS), étant de 70 : 30 à 10 : 90.

6. Agent particulaire absorbant l'eau selon l'une quelconque des revendications 1 à 5 comprenant en outre un agent réducteur inorganique.

7. Agent particulaire absorbant l'eau selon la revendication 6, l'agent réducteur inorganique contenant un atome de soufre réducteur.

8. Agent particulaire absorbant l'eau selon l'une quelconque des revendications 1 à 7 comprenant en outre des particules fines inorganiques insolubles dans l'eau.

9. Agent particulaire absorbant l'eau selon l'une quelconque des revendications 1 à 8 comprenant en outre un agent désodorisant soluble dans l'eau.

10. Agent particulaire absorbant l'eau selon l'une quelconque des revendications 1 à 9 comprenant en outre un sel de métal polyvalent et/ou un polymère cationique.

11. Agent particulaire absorbant l'eau selon l'une quelconque des revendications 1 à 10 comprenant en outre un polyol.

**12.** Agent particulaire absorbant l'eau selon l'une quelconque des revendications 1 à 11 comprenant en outre un ion fer, une quantité de l'ion fer étant de 0,001 à 2 ppm.

**13.** Agent particulaire absorbant l'eau selon l'une quelconque des revendications 1 à 12, l'agent particulaire absorbant l'eau possédant : une capacité de rétention de fluide sans pression (CRC) incluant une correction de teneur en humidité pour être de 30 (g/g) ou plus par rapport à une solution saline physiologique, et une capacité de rétention de fluide sous pression à une pression de 2,06 kPa (AAP) incluant une correction de teneur en humidité pour être de 20 (g/g) ou plus par rapport à une solution saline physiologique.

**14.** Agent particulaire absorbant l'eau selon l'une quelconque des revendications 1 à 13, une teneur en humidité obtenue à partir d'une quantité de perte au séchage à 180 °C pendant 3 heures étant de 0,5 à 15 % en poids.

**15.** Procédé pour la production d'un agent particulaire absorbant l'eau comprenant une résine absorbant l'eau en tant que composant principal et un agent chélatant, l'agent absorbant l'eau contenant des particules dotées d'un diamètre de particule de moins de 300 μm et des particules dotées d'un diamètre de particule de 300 μm ou plus mais inférieur à 850 μm, comme spécifié par un tamis normalisé de la norme industrielle japonaise (JIS),

une quantité de l'agent chélatant inclus étant de 0,001 à 5 parties en poids par 100 parties en poids d'une résine absorbant l'eau en solide et satisfaisant la formule (1) :
[formule mathématique 3]

$$1,2 < C1/C2 < 4,0 \ … \ (1)$$

où, dans la formule (1), C1 indique une quantité (ppm) de l'agent chélatant présent dans les particules d'un diamètre de particule de moins de 300 μm, comme spécifié par un tamis normalisé de la JIS, parmi des composants de particules pour l'agent particulaire absorbant l'eau, et C2 indique une quantité (ppm) de l'agent chélatant présent dans les particules d'un diamètre de particule de 300 μm ou plus mais inférieur à 850 μm, comme spécifié par un tamis normalisé de la JIS, parmi des composants de particules pour l'agent particulaire absorbant l'eau ; et

le procédé comprenant l'un parmi les a) et b) suivants :

a) les étapes de :

ajout d'un agent chélatant à une résine réticulée en surface absorbant l'eau pour obtenir un granulé de résine absorbant l'eau, dont le diamètre moyen de particule en poids (D50) est augmenté jusqu'à 1,01 à 10 fois par rapport au diamètre moyen de particule en poids avant l'ajout de l'agent chélatant ; et
concassage du granulé de résine absorbant l'eau et réduction du diamètre moyen de particule en poids (D50) de 5 % ou plus pour obtenir un agent particulaire absorbant l'eau ; et

b) les étapes de :

dimensionnement d'une résine réticulée en surface absorbant l'eau en une pluralité de tailles différentes de particules ;
ajout d'un agent chélatant selon la pluralité des tailles différentes de particules et
re-mélange d'une pluralité des résines absorbant l'eau, auxquelles ledit agent chélatant a été ajouté, pour obtenir un agent particulaire absorbant l'eau.

**16.** Procédé selon la revendication 15, l'agent chélatant dans un état en cours de dissolution et/ou de dispersion dans un solvant mixte contenant de l'eau et un solvant organique hydrophile étant ajouté aux particules de résine absorbant l'eau pour obtenir un agent particulaire absorbant l'eau.

**17.** Procédé selon la revendication 16, le solvant organique hydrophile étant un polyol.

**18.** Article absorbant comprenant un agent particulaire absorbant l'eau comme décrit dans l'une quelconque des revendications 1 à 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006033477 A
- US 4959060 A
- US 4863989 A
- EP 1477349 A
- US 4972019 A
- JP 63153060 A
- JP 63127754 A
- US 6335406 B
- JP 2000038407 A
- JP H11246674 A
- JP H11315148 A
- JP H11315147 A
- US 7473470 B
- JP 2000007790 A
- JP 3107873 B
- JP 2003206381 A
- US 7737231 B
- JP 2001234087 A
- EP 1645596 A
- EP 2112172 A
- EP 2163302 A
- WO 2011040530 A
- US 8182916 B

- US 5599335 A
- US 7638570 B
- US 20080161512
- US 2009123197 A
- US 20080194863
- US 6241928 B
- US 2005215734
- US 7265190 B
- US 6987151 B
- US 6710141 B
- US 4893999 A
- WO 2011126079 A
- WO 2006100300 A
- WO 2011025012 A
- WO 2011025013 A
- WO 2011111657 A
- US 7183456 B
- WO 2005075070 A
- WO 2008090961 A
- US 2006204755
- US 5669894 A
- JP 9235378 A
- US 7960269 B

**Non-patent literature cited in the description**

- *Chem. Pharm. Bull.,* 1987, vol. 29, 3755-3757